(19) Europäisches Patentamt
European Patent Office
Office européen des brevets

(11) Publication number: **0 584 797 A2**

# (12) EUROPEAN PATENT APPLICATION

(21) Application number: **93113515.6**

(22) Date of filing: **24.08.93**

(51) Int. Cl.$^5$: **C07D 501/36**, A61K 31/545

(30) Priority: **24.08.92 KR 1517692**

(43) Date of publication of application:
**02.03.94 Bulletin 94/09**

(84) Designated Contracting States:
**AT BE CH DE DK ES FR GB GR IE IT LI LU MC NL PT SE**

(71) Applicant: **LUCKY LTD.**
**20, Yoido-dong,**
**Yongdungpo-ku**
**Seoul 150-721(KR)**

(72) Inventor: **Kim, Won-Sub**
**Lucky Apt. A-303,**
**386-4, Doryong-Dong**
**Youseong-Ku, Daejeon(KR)**
Inventor: **Lim, Jong-Chan**
**Lucky Apt. A-104,**
**386-4, Doryong-Dong**
**Youseong-Ku, Daejeon(KR)**
Inventor: **Bang, Chan-Sik**
**Lucky dorm 210,**
**386-4, Doryong-Dong**
**Youseong-Ku, Daejeon(KR)**
Inventor: **Yeo, Jae-Hong**
**118-1, Shinseong-Dong**
**Youseong-Ku, Daejeon(KR)**
Inventor: **Kim, Yong-Zu**
**Lucky Hana APT. 102-401, 153**
**Shinsung-dong**
**Youseong-Ku, Daejeon(KR)**
Inventor: **Oh, Hun-Seung**
**Lucky Hana APT. 102-103, 153**
**Shinsung-dong**
**Youseong-Ku, Daejeon(KR)**
Inventor: **Son, Heui-Sung**
**Lucky Dorm 511,**
**386-1, Doryong-Dong**
**Youseong-Ku, Daejeon(KR)**
Inventor: **Kim, Mi-Rry**
**125-4, Nongsung-1-Dong**
**Seo-Ku, Gwangju(KR)**
Inventor: **Seo, Mie-Kyeong**
**Lucky dorm 426,**
**386-1, Doryong-Dong**
**Youseong-Ku, Daejeon(KR)**
Inventor: **Yim, Hyeon-Joo**
**357-13, Dae-dong**
**Dong-Ku, Daejeon(KR)**
Inventor: **Kim, Yun-Sik**
**Sangka Apt. Na-1001,**
**34-3, Shinchon-dong**
**Changwon(KR)**
Inventor: **Nahm, Kee-Pyung**
**Lucky Apt. 6-102,**
**381-42, Doryong-Dong**
**Youseong-Ku, Daejeon(KR)**

(74) Representative: **Patentanwälte Grünecker, Kinkeldey, Stockmair & Partner**
**Maximilianstrasse 58**
**D-80538 München (DE)**

## (54) Novel cephalosporin compounds and processes for the preparation thereof.

(57) The present invention relates to novel cephalosporin compounds, pharmaceutically acceptable non-toxic salts, physiologically hydrolyzable esters, hydrates and solvates and isomers thereof which possess potent and broad antibacterial activities. The compounds of the present invention have a (1,5,6-substituted-4-aminopyrimidinium-2-yl)thiomethyl group in 3-position of the cephem nucleus and is specifically represented by the following formula(I):

EP 0 584 797 A2

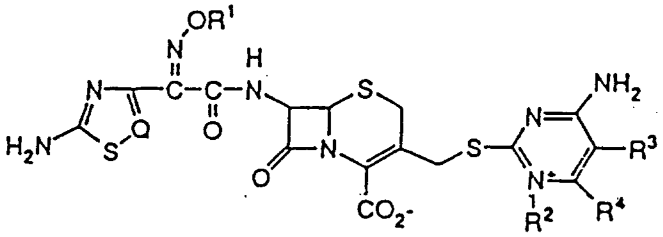

( I )

Field of the Invention

The present invention relates to novel cephalosporin compounds, pharmaceutically acceptable non-toxic salts, physiologically hydrolyzable esters, hydrates and solvates, and isomers thereof, which possess potent and broad spectrum of antibacterial activities. The invention also relates to processes for preparing these compounds and to pharmaceutical compositions containing same as active ingredients.

Description of the Prior Art

Antibiotics of cephalosporin series are widely used in therapy for the treatment of diseases which are caused by general pathogenic bacteria in human beings and animals. It has been known that such antibiotics are useful for the treatment of diseases caused by bacteria exhibiting resistance to other antibiotics, e.g., penicillin-resistant bacteria; and also for the treatment of penicillin-sensitive patients.

In most circumstances, it is desirable to employ antibiotics possessed with a wide range of antibacterial activities, e.g., against both Gram-positive and Gram-negative bacteria. It is well known that the activity of a cephalosporin compound depends upon the substituent on the 3- or 7-position of the cephem ring. In this regard, there are many studies made in developing a variety of cephalosporin antibiotics with such broadspectrum of antibiotic activities by introducing a 7-$\beta$ acylamido group and various substituents on the 3-position of the cephem ring.

For example, Japanese Laid-open Patent Publication No. 54-9296 discloses a cephalosporin derivative having a substituent, on the 3-position of the cephem ring, of the folling formula(A):

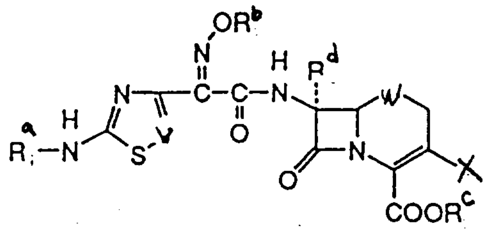

(A)

wherein:

X Represents a hydrogen, an optionally substituted alkyloxy or alkenyloxy, halogen or $-CH_2Y$ group where Y represents a hydrogen or halogen or moiety of a nucleophilic compound including -SR where R represents an optionally substituted 5- to 8- membered heterocyclic ring.

French Patent No. 2,426,694 provides a 7-[(2-aminothiazol-4-yl)-2-oxyiminoacetamido]cephem derivative having certain substituents, in the 7$\beta$- and 3-positions, of the formula(B):

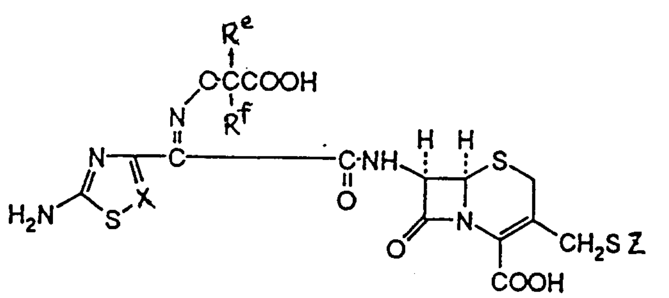

(B)

wherein:

$R^e$ and $R^f$ form a $C_{3-7}$ cycloalkylidene group with the carbon atoms to which they are linked; and

Z represents a 5- or 6-membered heterocyclic ring, linked to a carbon atom, which contains one or more nitrogen atom, and may contain one or more sulfur atom and/or may be substituted with a $C_{1-4}$ alkyl group.

European Patent Application No. 87304896.1 discloses a cephalosporin compound of the formula(C):

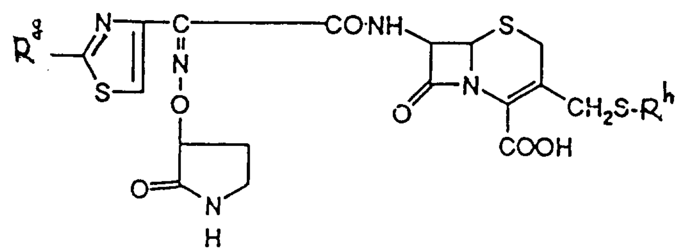

(C)

wherein:

$R^h$ represents

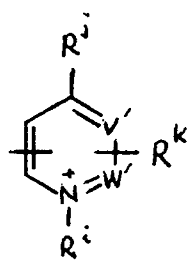

(C−1)

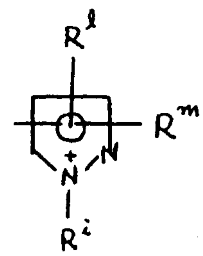

(C−2)

$R^i$ represents a lower alkenyl, optionally substituted 5- or 6-membered nitrogen-containing heterocyclic ring group, acylamino or optionally substituted lower alkyl group;

$R^j$ and $R^k$, which may be the same or different, are each independently a hydrogen, lower alkyl, amino, acylamino, carboxyl, carbamoyl, thiocarbamoyl or lower alkoxycarbonyl group;

$R^l$ and $R^m$ are each independently a lower alkyl group; and

V' and W', which may be the same or different, are each independently -CH= or -N= group.

Further, cephalosporin compounds having various 4,6-diaminopyrimidinium moiety on the 3-position, with improved properties with respect to certain microorganisms, especially against Gram-negative bacteria, of the following formula(D) are disclosed in Korean Patent Nos. 47728, 47754, 47755 and 47756:

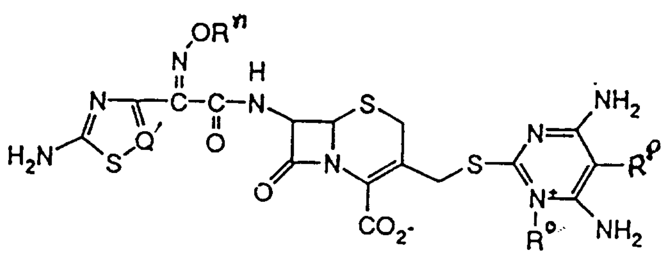

(D)

wherein:

$R^n$ is a $C_{1-4}$ alkyl, $C_{3-4}$ alkenyl, $C_{3-4}$ alkynyl or $C(R^A)(R^B)COOH$;

$R^o$ is a $C_{1-4}$ alkyl, $C_{3-4}$ alkenyl, $C_{3-4}$ alkynyl, or optionally substituted amino or phenyl group;

$R^p$ represents a hydrogen or $C_{1-4}$ alkyl group; and

Q' is CH or N.

However, none of the above-mentioned prior art literature has disclosed or taught the possibility of employing a (1,5,6-substituted-4-aminopyrimidinium-2-yl) thiomethyl group as a substituent in the 3-position of the cephem nucleus.

Summary of the Invention

The present inventors have stuided for a long time in search for a cephalosporin compound which has a broad spectrum of antibiotic activities. As a result, the present inventors have discovered that cephalosporin compouns with a (1,5,6-substituted-4-aminopyrimidinium-2-yl)thiomethyl group in the 3-position and a certain group in the 7-$\beta$-position of the cephem nucleus exhibit strong antibacterial activities in a broad spectrum.

Accordingly, the primary object of the present invention is to provide said novel cephalosporin compounds.

It is another object of the present invention to provide processes for preparing such compounds.

It is still another object of the present invention to provide pharmaceutical compositions containing same.

In accordance with one aspect of the present invention, there is provided with novel cephalosporin compounds of formula(I):

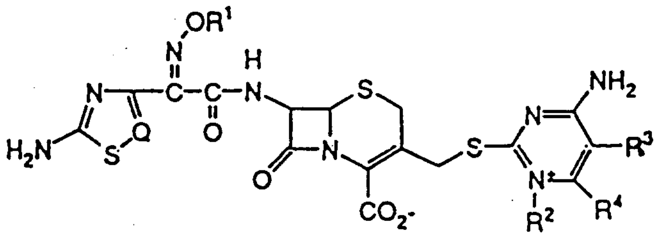

(I)

wherein:

| | |
|---|---|
| $R^1$ | is a hydrogen, a $C_{1-4}$ alkyl, $C_{3-4}$ alkenyl, $C_{3-4}$ alkynyl or $-C(R^A)(R^B)COOH$ group wherein $R^A$ and $R^B$ are independently a hydrogen or a $C_{1-4}$ alkyl group, or form a $C_{3-7}$ cycloalkyl group together with the carbon atoms to which they are attached ; |
| $R^2$ | is a $C_{1-4}$ alkyl, carboxymethyl, hydroxyethyl or amino group; |
| $R^3$ and $R^4$ | are independently a hydrogen or a $C_{1-4}$ alkyl, amino which is optionally substituted, benzyl, $C_{1-4}$ alkoxy or carboxymethoxy group; or form a cyclic ring, provided that $R^3$ is not a hydrogen or a $C_{1-4}$ alkyl group when $R^4$ is an unsubstituted amino group; and |
| Q | is CH or N. |

In accordance with another aspect of the present invention, there is provided with processes for preparing the cephalosporin compounds of formula(I).

In accordance with a further aspect of the present invention, there is provided with pharmaceutically acceptable non-toxic salts, physiologically hydrolysable esters, hydrates and solvates of the compounds of formula(I) and processes for preparing these compounds.

In accordance with a still further aspect of the present invention, there is provided with pharmaceutical compositions comprising one or more of the cephalosporin compounds represented by formula(I) and their afore-mentioned derivatives as active ingredients and their pharmaceutically acceptable carriers.

Detailed Description of the Invention

The novel cephalosporin compounds of formula(I) include both syn isomers and mixtures of syn and anti isomers, with respect to the radical, $-O-C(R^A)(R^B)-COOH$, which mixtures contain at least 90% of the syn isomer. The compounds of formula(I) also include the diastereomeric isomers and mixtures thereof when $R^A$ and $R^B$ are different from each other.

In addition, the compounds of formula(I) in accordance with the present invention may exist in tautomeric forms and such tautomers are also included within the scope of the invention. Namely, when $Q_1$ of formula(I) is CH, the aminothiazolyl group undergoes tautomerism to form an iminothiasolinyl group, its tautomer, as follows:

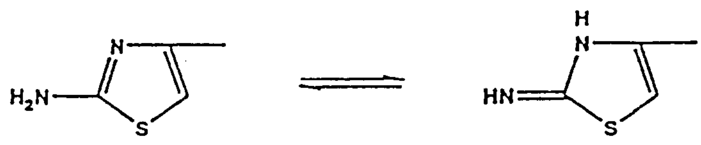

When $Q_1$ of formula(I) is N, the aminothiadiazolyl group forms the iminothiadiazolinyl groups, its tautomers, as follows:

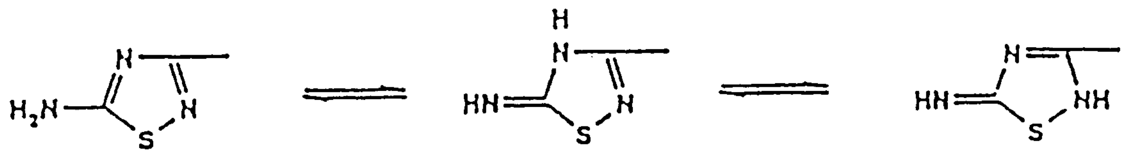

Among the compounds of the present invention, preferred compounds are those wherein: both $R^3$ and $R^4$ are a hydrogen or an amino group. More preferred compounds are those wherein: $R^1$ is a $-C(R^A)(R^B)-COOH$ group wherein $R^A$ and $R^B$ are independently a hydrogen or a methyl or ethyl group, or form an unsubstituted $C_{4-6}$ cycloalkyl group together with the carbon atoms to which they are attached; $R^2$ is a $C_{1-4}$ alkyl, preferably methyl or ethyl group, or amino group; both $R^3$ and $R^4$ are a hydrogen or an amino group; and Q is CH.

Exemplary compounds of formula(I) are listed in Table 1.

*Table 1. Examplary Compounds of formula(I)*

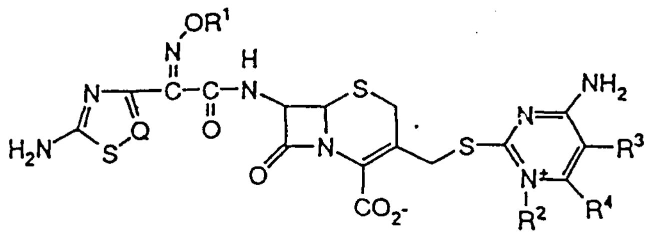

( I )

| Example No. | Q | $R^1$ | $R^2$ | $R^3$ | $R^4$ |
|---|---|---|---|---|---|
| 1 | CH | 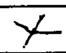 $CO_2H$ | $CH_3$ | $NH_2$ | $NH_2$ |
| 2 | CH | 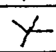 $CO_2H$ | $CH_3$ | H | H |
| 3 | CH | $CH_2CH_3$ | $CH_3$ | $NH_2$ | $NH_2$ |
| 4 | CH | $CH_2CH_3$ | $CH_3$ | H | H |
| 5 | N | $CH_2CH_3$ | $CH_3$ | $NH_2$ | $NH_2$ |
| 6 | N | $CH_2CH_3$ | $CH_3$ | H | H |
| 7 | CH | $CH_3$ | $CH_3$ | $NH_2$ | $NH_2$ |
| 8 | CH | $CH_3$ | $CH_3$ | H | H |
| 9 | CH | H | $CH_3$ | $NH_2$ | $NH_2$ |
| 10 | CH | H | $CH_3$ | H | H |
| 11 | CH | 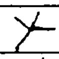 $CO_2H$ | $CH_2CH_3$ | $NH_2$ | $NH_2$ |
| 12 | CH | 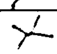 $CO_2H$ | $CH_2CH_3$ | H | H |
| 13 | CH | $CH_2CH_3$ | $CH_2CH_3$ | $NH_2$ | $NH_2$ |
| 14 | CH | $CH_2CH_3$ | $CH_2CH_3$ | H | H |
| 15 | N | $CH_2CH_3$ | $CH_2CH_3$ | $NH_2$ | $NH_2$ |
| 16 | N | $CH_2CH_3$ | $CH_2CH_3$ | H | H |
| 17 | CH | 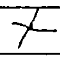 $CO_2H$ | $CH_3$ | H | $OCH_3$ |
| 18 | CH | $CH_2CH_3$ | $CH_3$ | H | $OCH_3$ |
| 19 | CH | $CH_2CH_3$ | $CH_3$ | H | $OCH_2CO_2H$ |
| 20 | CH | 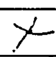 $CO_2H$ | $CH_3$ | H | 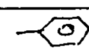 |
| 21 | CH | 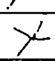 $CO_2H$ | $CH_3$ | 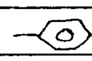 | H |
| 22 | CH | $CH_3$ | $CH_2CO_2H$ | H | H |
| 23 | CH | $CH_2CH_3$ | $CH_2CO_2H$ | H | H |
| 24 | CH | 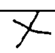 $CO_2H$ | $CH_2CO_2H$ | H | H |

Table 1(continued)

| Example No. | Q | $R^1$ | $R^2$ | $R^3$ | $R^4$ |
|---|---|---|---|---|---|
| 25 | CH | $CH_2CH_3$ | $CH_3$ | $CO_2H$ | H |
| 26 | CH | $CH_2CH_3$ | $CH_3$ | $NH_2$ | H |
| 27 | CH | 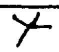 $CO_2H$ | $CH_3$ | $NH_2$ | H |
| 28 | CH | 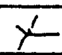 $CO_2H$ | $CH_3$ | 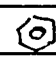 | |
| 29 | CH | $CH_2CH_3$ | $CH_3$ | 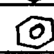 | |
| 30 | CH | 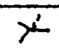 $CO_2H$ | $CH_3$ | 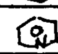 | |
| 31 | CH | $CH_2CH_3$ | $CH_3$ | 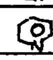 | |
| 32 | CH | 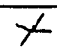 $CO_2H$ | $CH_3$ | 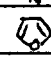 | |
| 33 | CH | $CH_2CH_3$ | $CH_3$ | 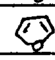 | |
| 34 | CH | 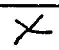 $CO_2H$ | $CH_3$ | $CH_2$ 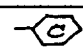 | H |
| 35 | CH | 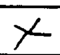 $CO_2H$ | $CH_3$ | H | $NHCH_3$ |
| 36 | CH | $CH_2CH_3$ | $CH_3$ | H | $NHCH_3$ |
| 37 | CH | 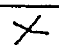 $CO_2H$ | $CH_3$ | H | $NHCH_2CH_3$ |
| 38 | CH | $CH_2CH_3$ | $CH_3$ | H | $NHCH_2CH_3$ |
| 39 | CH | 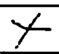 $CO_2H$ | $CH_3$ | $CO_2H$ | H |
| 40 | CH | 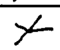 $CO_2H$ | $CH_3$ | $CONH_2$ | H |
| 41 | CH | $CH_2CH_3$ | $CH_3$ | $CONH_2$ | H |
| 42 | CH | $CH_2CH_3$ | $NH_2$ | H | H |
| 43 | CH | 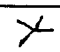 $CO_2H$ | $NH_2$ | H | H |
| 44 | CH | $CH_3$ | $NH_2$ | H | H |
| 45 | N | $CH_2CH_3$ | $NH_2$ | H | H |
| 46 | CH | $CH_2CH_3$ | $NH_2$ | $NH_2$ | H |
| 47 | CH | 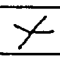 $CO_2H$ | $NH_2$ | $NH_2$ | H |
| 48 | CH | $CH_3$ | $NH_2$ | $NH_2$ | H |
| 49 | N | $CH_2CH_3$ | $NH_2$ | $NH_2$ | H |
| 50 | CH | 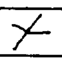 $CO_2H$ | $CH_3$ | H | $N(CH_3)_2$ |
| 51 | CH | $CH_3$ | $CH_3$ | H | $N(CH_3)_2$ |
| 52 | CH | $CH_2CH_3$ | $CH_3$ | H | $N(CH_3)_2$ |
| 53 | CH | 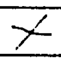 $CO_2H$ | $NH_2$ | $NH_2$ | $NH_2$ |

Table 1 (continued)

| Example No. | Q | $R^1$ | $R^2$ | $R^3$ | $R^4$ |
|---|---|---|---|---|---|
| 54 | CH | $CH_3$ | $NH_2$ | $NH_2$ | $NH_2$ |
| 55 | CH | $CH_2CH_3$ | $NH_2$ | $NH_2$ | $NH_2$ |
| 56 | N | $CH_2CH_3$ | $NH_2$ | $NH_2$ | $NH_2$ |
| 57 | CH | 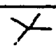 $CO_2H$ | $NH_2$ | $CH_3$ | H |
| 58 | CH | 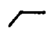 $CO_2H$ | $NH_2$ | $CH_3$ | H |
| 59 | CH | $CH_3$ | $NH_2$ | $CH_3$ | H |
| 60 | CH | $CH_2CH_3$ | $NH_2$ | $CH_3$ | H |
| 61 | N | $CH_2CH_3$ | $NH_2$ | $CH_3$ | H |
| 62 | CH | 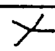 $CO_2H$ | $NH_2$ | $CH_2CH_3$ | H |
| 63 | CH | $CH_2CH_3$ | $NH_2$ | $CH_2CH_3$ | H |
| 64 | CH | $CH_3$ | $NH_2$ | $CH_2CH_3$ | H |
| 65 | CH | 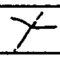 $CO_2H$ | $NH_2$ | H | $NHCH_3$ |
| 66 | CH | $CH_2CH_3$ | $NH_2$ | H | $NHCH_3$ |
| 67 | CH | $CH_3$ | $NH_2$ | H | $NHCH_3$ |
| 68 | N | $CH_2CH_3$ | $NH_2$ | H | $NHCH_3$ |
| 69 | CH | 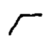 $CO_2H$ | $NH_2$ | H | $NHCH_3$ |
| 70 | CH | 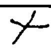 $CO_2H$ | $NH_2$ | $CH_3$ | $NHCH_3$ |
| 71 | CH | $CH_2CH_3$ | $NH_2$ | $CH_3$ | $NHCH_3$ |
| 72 | CH | $CH_3$ | $NH_2$ | $CH_3$ | $NHCH_3$ |
| 73 | N | $CH_2CH_3$ | $NH_2$ | $CH_3$ | $NHCH_3$ |
| 74 | CH | 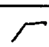 $CO_2H$ | $NH_2$ | $CH_3$ | $NHCH_3$ |
| 75 | CH | 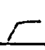 $CO_2H$ | $NH_2$ | H | H |
| 76 | CH | 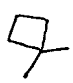 $CO_2H$ | $NH_2$ | H | H |
| 77 | CH | 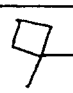 $CO_2H$ | $CH_3$ | H | H |
| 78 | CH | 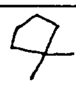 $CO_2H$ | $NH_2$ | H | H |
| 79 | CH | 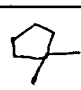 $CO_2H$ | $CH_3$ | H | H |

Table 1(continued)

| Example NO. | Q | $R^1$ | $R^2$ | $R^3$ | $R^4$ |
|---|---|---|---|---|---|
| 80 | CH | 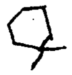 $CO_2H$ | $NH_2$ | H | H |
| 81 | CH | 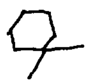 $CO_2H$ | $CH_3$ | H | H |
| 82 | CH | 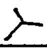 $CO_2H$ | $CH_3$ | $NH_2$ | $NH_2$ |
| 83 | CH | 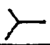 $CO_2H$ | $CH_3$ | H | H |
| 84 | CH | 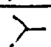 $CO_2H$ | $NH_2$ | H | H |
| 85 | CH | 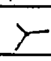 $CO_2H$ | $NH_2$ | $CH_3$ | H |
| 86 | CH | 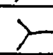 $CO_2H$ | $NH_2$ | $CH_3$ | $NHCH_3$ |
| 87 | CH | 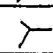 $CO_2H$ | $NH_2$ | H | $NHCH_3$ |
| 88 | CH | 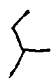 $CO_2H$ | $NH_2$ | H | H |
| 89 | CH | 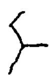 $CO_2H$ | $CH_3$ | H | H |
| 90 | CH | 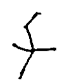 $CO_2H$ | $NH_2$ | H | H |
| 91 | CH | 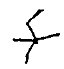 $CO_2H$ | $CH_3$ | H | H |

Furthermore, the present invention encompasses, within its scope, those pharmaceutically acceptable non-toxic salts, physiologically hydrolyzable esters, solvates and hydrates of the compounds of formula(I). Suitable pharmaceutically acceptable salts of the cephalosporin compounds(I) are conventional non-toxic salts and may include: inorganic acid salts(e.g., hydrochloride, hydrobromide, sulfate, phosphate, etc.); organic carboxylic and sulfonic acid salts(e.g., formate, trifluoroacetate, citrate, acetate, maleate, tartrate, oxalate, succinate, benzoate, fumarate, mandelate, ascorbate, malate, methanesulfonate, para-toluenesulfonate, etc.); and also, depending on $R^1$, inorganic and organic base salts such as salts with alkali metal hydroxides(e.g., sodium hydroxide, potassium hydroxide, etc.), alkaline earth metal hydroxides (e.g., calcium hydroxide etc., ), sodium bicarbonate, potassium bicarbonate, sodium carbonate, potassium carbonate and calcium carbonate, and aminoic acid salts.

Above pharmaceutically acceptable non-toxic salts may be prepared in accordance with known methods, e.g., by reacting the compounds of the formula(I) with one to four equivalents of corresponding acids or bases to form the salts mentioned above in the presence of a solvent which may be water, or a mixture of water and water-miscible solvent(e.g., methanol, ethanol, acetonitrile, acetone, etc.).

The physiologically hydrolyzable esters of the compounds(I) may include, for example, indanyl, phthalidyl, methoxymethyl, pivaloyloxymethyl, glycyloxymethyl, phenylglycyloxymethyl and 5-methyl-2-oxo-1,3-dioxolan-4-yl esters, and other physiologically hydrolyzable esters which have been widely used in the penicillin and cephalosporin antibiotics art.

These esters can be prepared in accordance with known methods, e.g., by reacting the compounds of formula(I) with a corresponding alkyl halide(e.g., methoxymethyl chloride) in the presence of a base(e.g., triethylamine, pyridine or sodium bicarbonate).

Exemplary solvates of the cephalosporin compounds of formula(I) may include those solvates with water-miscible solvents, e.g., methanol, ethanol, acetone and acetonitrile, preferably, ethanol.

The present invention also includes within its scope pharmaceutical compositions comprising one or more of the compounds(I) and their above-mentioned derivatives as active ingredients, in association with pharmaceutically acceptable carriers, excipients or other additives, if necessary.

The pharmaceutical compositions of the invention may be formulated for administration in unit dose or multi-dose containers. The compositions may take various forms such as solution, suspension or emulsion in an oily or aqueous vehicle, which can contain conventional additives such as a dispersant, suspending agent, stabilizer and the like. Alternatively, the active ingredient may be formulated into a dried powder that can be normally dissolved in an aqueous solution of sterile pyrogen-free water before use. The compositions may be also formulated into suppositories containing conventional suppository bases such as cocoa butter or other glycerides.

The pharmaceutical compositions in a unit dose form may preferably comprise about 50 to 1,500mg of the active ingredient, depending on the age and body weight of the patient, the nature and severity of the illness, and so on. In general, it has been shown advantageous to administer the active compounds in an amount ranging from 500 to 5,000mg per day in order to achieve the desired results, depending on the routes and frequency of administration. In case of intramuscular or intravenous administration for adult human treatment, the dosage of about 150 to 3,000mg per day is thought to be sufficient, although it may vary in case of treatment for specific infections caused by certain strains.

The compounds of the present invention, as described above, exhibit potent and broad antibacterial activities against Gram-positive bacteria and a variety of Gram-negative bacteria as well particularly against Pseudomonas. Also, these compounds have high stability to $\beta$-lactamases produced by a number of Gram-negative bacteria.

A compound of formula(I) may be prepared by reacting a compound of formula(II) with a compound of formula(III) in the presence of a solvent, and, if necessary, removing the amino or carboxylic acid protecting group or reducing $S \rightarrow (O)_n$:

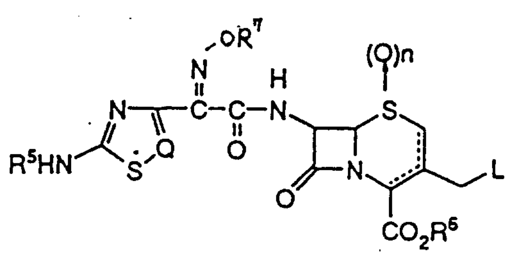

(II)

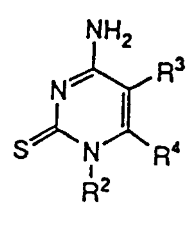

(III)

wherein:

| | |
|---|---|
| $R^2$, $R^3$, $R^4$ and Q | have the same meanings as defined before; |
| n | is 0 or 1; |
| $R^5$ | is a hydrogen or an amino protecting group; |
| $R^6$ | is a hydrogen or a carboxyl protecting group; |
| $R^7$ | is a hydrogen, a $C_{1-4}$ alkyl, $C_{3-4}$ alkenyl, $C_{3-4}$ alkynyl or $-C(R_A)(R_B)-COOR^C$ wherein $R^A$ and $R^B$, are independently a hydrogen or a $C_{1-4}$ alkyl group, or together with the carbon atoms to which they are attached, form a C3-7 cycloalkyl group; and $R^C$ is a hydrogen or a carboxylic protecting group; and |
| L | is a leaving group. |

The amino protecting group in $R^5$ above may be those groups which can be readily removed under the conventional mild conditions to form a free amino group and may include: acyl, substituted and unsubstituted aryl(lower)alkyl(e.g., benzyl, diphenylmethyl and triphenylmethyl), (lower)alkoxyaryl (e.g., 4-methoxybenzyl), halo(lower)alkyl(e.g., trichloromethyl and trichloroethyl), tetrahydropyranyl, substituted phenylthio, substituted alkylidene, substituted aralkylidene and substituted cycloalkylidene. The acyl group as an amino protecting group may, for example, $C_{1-6}$ alkanoyl(e.g., formyl and acetyl), $C_{2-6}$ alkoxycarbonyl(e.g., methoxycarbonyl and ethoxycarbonyl), (lower)alkanesulfonyl (e.g., methane-sulfonyl and ethanesulfonyl), and aryl(lower) alkoxycarbonyl (e.g., benzyloxycarbonyl), where the acyl group can be substituted with 1 to 3 substiutents such as a halogen, a hydroxy, cyano and nitro group. In addition, the amino protecting group may include the reaction products obtained from reacting amino groups with silane, boron or phosphorous compounds.

The carboxyl protecting group of $R^C$ or $R^6$ may be any one of those which can be readily removed under the conventional mild conditions to form a free carboxyl group and may include for example, (lower)-alkylesters(e.g., methyl ester and tert-butyl ester), (lower)alkenylesters(e.g., vinylester and allylester), (lower)alkoxy(lower)alkylesters(e.g., methoxymethylester), (lower)alkylthio(lower)alkylesters(e.g., methylthiomethylester), halo(lower)alkylesters(e.g., 2,2,2-trichloroethylester), substituted and unsubstituted aralkyl esters(e.g., benzylester and p-nitrobenzyl ester), (lower) aralkoxy esters(e.g., p-methoxybenzyl ester) and silyl esters. The amino or carboxyl protecting group can be properly selected after considering the chemical property of the desired compound(I).

The leaving group L in formula(II) may include, for example, a halogen such as chlorine and fluorine, a (lower) alkanoyloxy group such as acetoxy, a (lower)alkanesulfonyloxy group such as methanesulfonyloxy, an arenesulfonyloxy group such as p-toluenesulfonyloxy, an alkoxycarbonyloxy group and the like.

The term "lower" as used hereinabove and elsewhere in this specification, for example, in reference to "lower alkyl", encompasses those compounds having 1 to 6 carbon atoms, more preferably, 1 to 4 carbon atoms.

The starting materials of the compounds(II) are known intermediates conventionally employed for the preparation of cephalosporin compounds. The dotted line of formula(II) represents a single or double bond; and, therefore, the compounds of formula(II) may be the compounds of formula (II-a), or the compounds of formula(II-b), or mixtures thereof:

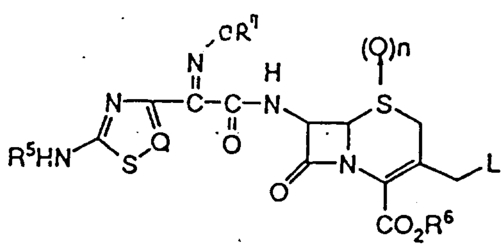

(II-a)

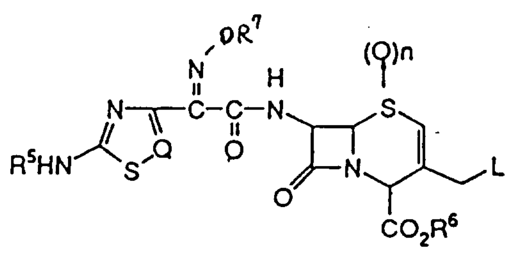

(II-b)

wherein n, $R^5$, $R^6$, $R^7$, L and Q have the same meanings as defined before.

The compounds of formula(II) may be prepared by activating the compounds of formula(IV) or salts thereof with an acylating agent and then reacting with the compounds of formula(V) in accordance with the following scheme(A).

Scheme A

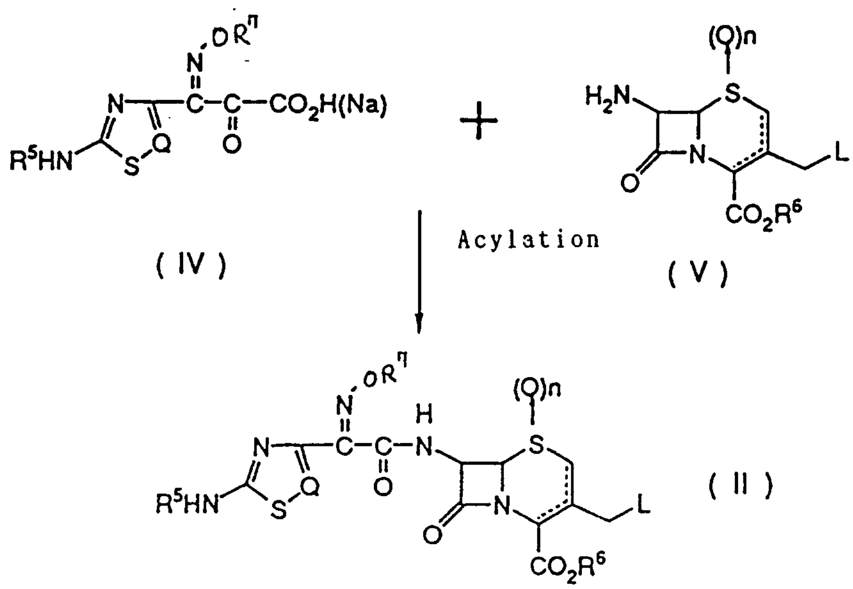


( IV ) ( V ) ( II )

wherein:

n, $R^5$, $R^6$, $R^7$, L and Q have the same meanings as defined previously.

The dotted line of formula(V) represents a single or double bond; and therefore, the compounds of formula(V) may be the compounds of formula(V-a) or the compounds of formula(V-b), or mixtures thereof:

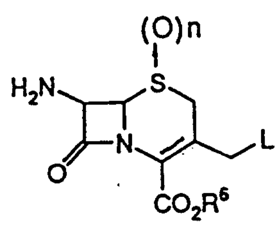

( V - a )

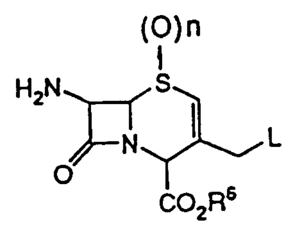

( V - b )

The acylated derivative from the compound of formula(IV) may be an acid chloride, anhydrous acid, mixed anhydrous acid (preferably, anhydrous acid formed with methyl chloroformate, mesitylenesulfonyl chloride, p-toluenesulfonyl chloride or chlorophosphate) or activated ester (preferably an ester formed by reaction with N-hydroxy benzotriazole in the presence of a condensing agent, e.g., dicyclohexyl carbodiimide). The acylation may be conducted by using a free acid of the compound(IV) in the presence of a condensing agent, e.g., dicyclohexyl carbodiimide or carbonyl diimidazole. Further, the acylation may be conventionally conducted in the presence of an organic base, e.g., a tertiary amine(prferably, triethyl amine), diethylaniline and pyridine, or an inorganic base, e.g., sodium bicarbonate and sodium carbonate, and a solvent, e.g., a halogenated hydrocarbon (e.g., methylene chloride and chloroform), tetrahydrofuran, acetonitrile, dimethyl formamide, dimethyl acetamide and mixtures thereof and an aqueous mixture thereof.

The acylation may be conducted at a temperature ranging from -50 ° C to 50 ° C, preferably from -30 ° C to 20 ° C, and the acylating agent may be used in a stoichiometric amount, or an excess(1.05 to 1.2 equivalents) thereof, based on the compound of formula(V).

In order to prepare the compound of formula(I), amino or carboxyl protecting groups of formula(II) can be readily removed by any of the conventional deprotecting methods which are well known in the field of cephalosporin antibiotics. For example, acid- or base-hydrolysis or reduction is generally applicable. For example, when the compound of formula(II) comprises an amido group as a protecting group, the

compound may be subjected to an aminohalogenation, aminoetherification and hydrolysis procedure. The acid-hydrolysis is suitable for removing a tri(di)phenylmethyl or alkoxycarbonyl group; and may be conducted by employing an organic acid, e.g., formic acid, trifluoroacetic acid and p-toluenesulfonic acid, or an inorganic acid, e.g., hydrochloric acid.

The compounds of formula(III) used in the present invention are novel materials. The processes for the preparation of the compound(III) are specifically disclosed in the following Preparation Examples.

The reaction for introducing the compound(III) into the 3-position of the compound(II) to prepare the compound(I) is carried out in the presence of a solvent such as a polar solvent, e.g., dimethylformamide, dimethylsulfoxide, dimethylacetamide and the like, wherein the temperature may range from 10 to 80°C, more preferably from 20 to 40°C; and the compounds of the formula(III) are used in a stoichiometric amount, or in an 0.8 to 0.9 molar equivalent, based on the compounds of formula(II).

Reduction of S-oxide can be conventionally carried out, for example, by adding potassium iodide and acetyl chloride to the reactants, followed by quenching the reaction mixture with sodium m-bisulfite.

The separation and purification of the compounds(I) can be carried out by using a conventional method such as recrystallization, column chromatography over silica gel or ion-exchange chromatography.

The following Preparation Examples and Examples illustrate how some of the starting materials of formulae(II) and (III) and of the compounds of formula(I) can be prepared.

Preparation Example 1: Synthesis of 4,5,6-triamino-1-methyl-2-pryrimidinethione

Step 1) Synthesis of 4,5,6-triamino-2-methylthiopyrimidine

To a solution of 245g of 4,6-diamino-2-methylthiopyrimidine dissolved in 800 ml of acetic acid was added 250 ml of water. The reaction solution was cooled to 10°C and thereto was added a solution of 119g of $NaNO_2$ dissolved in 250 ml of water while maintaining the temperature at 10°C. The reaction mixture was stirred at room temperature for 2 hours, filtered and dried to obtain 4,6-diamino-5-nitroso-2-methylthiopyrimidine, which was then dissolved in 1l of water. The resulting solution was warmed to a temperature ranging from 60 to 65°C and thereto was added $Na_2S_2O_4$ until color of the solution became disappeared. The resultant clear solution was adjusted to pH 9 and cooled to produce precipitates, which were filtered and dried to obtain 98.4g of the title compound.

m.p.: 208°C

NMR($\delta$, DMSO-$d_6$): 5.68(s, 4H), 3.58(s, 2H), 2.31(s, 3H)

Step 2) Synthesis of 4,5,6-triamino-1-methyl-2-pyrimidinethione

98.4g(0.58mole) of the compound obtained in step 1 was dissolved in 600 ml of tetrahydrofuran, and thereto was added 163.4g (1.15mole) of methyl iodide. The reaction mixture was heated under reflux for 24 hours and cooled to produce precipitates, which were filtered and dried to obtain 77.4g of 4,5,6-triamino-1-methyl-2-methylthiopyrimidinium iodide. To the compound thus obtained were added 700 ml of methanol and then 34.5g of $NaSH \cdot 3H_2O$, and the resultant solution was heated under reflux for 12 hours and cooled to about 10°C to produce precipitates, which were filtered, washed with water and with acetone and dried to obtain 20.6g of the title compound.

m.p.: 192°C

NMR($\delta$, DMSO-$d_6$): 6.45(s, 2H), 6.36(s, 2H), 3.78(s, 3H), 3.34(s, 2H)

MS(EI, M): 171, 156, 138

Preparation Example 2: Synthesis of 4-amino-1-methyl-2-pyrimidinethione

To a solution of 97.12g(1 mole) of 3-ethoxy acrylonitrile dissolved in 200 ml of dry ethanol were added 145g of sodium ethoxide and 90.15g of N-methyl thiourea. The reaction mixture was heated under reflux for 12 hours and cooled to produce precipitates, which were filtered, washed with water and with acetone and dried to obtain 86g of the title compound.

m.p.: 98°C

NMR($\delta$, DMSO-$d_6$): 7.86(d, 1H), 7.57(bs, 2H), 6.00(d, 1H), 3.61(s, 3H)

MS(EI, M): 141, 126

Preparation Example 3: Synthesis of 4,5,6-triamino-1-ethyl-2-pyrimidinethione

The same procedures as described in step 2 of Preparation 1 above were repeated except that methyl iodide was substituted with 320g(2 mole) of ethyl iodide to obtain 26.84g of the title compound.

m.p.: 220 ° C

NMR(δ, DMSO-$d_6$): 6.54(s, 2H), 6.30(s, 2H), 6.58(q, 2H), 3.30(s, 2H), 1.23(t, 3H)

MS(EI, M): 185, 156

Preparation Example 4: Synthesis of 4-amino-1-ethyl-2-pyrimidinethione

The same procedures as described in Preparation Example 2 above were repeated except that N-methyl thiourea was substituted with N-ethyl thiourea to obtain 75g of the title compound.

m.p.: 187 ° C

NMR(δ, DMSO-$d_6$): 7.78(d, 1H), 7.60(bd, 2H), 6.04(q, 1H), 3.54(d, 1H), 3.54(q, 2H), 1.24(t, 3H)

MS(EI, M): 155, 126

Preparation Example 5: Synthesis of 4-amino-6-methoxy-1-methyl-2-pyrimidinethione

A mixture Of 17.1g of 4-amino-1-methyl-2-methylthiopyrimidin-6-one and 15g of methyl iodide with 150 ml of tetrahydrofuran was heated under reflux for 18 hours and cooled to produce precipitates, which were filtered, dried and dissolved in 50 ml of methanol. Thereto was added 8g of $NaSH•3H_2O$, and the resultant solution was heated under reflux until no bubbles were generated, and cooled to room temperature to produce precipitates, which were filtered, washed with 20 ml of water and with 20 ml of acetone and dried to obtain 4.8g of the title comound.

m.p.: 202 ° C

NMR(δ, DMSO-$d_6$): 3.81(s, 3H), 3.87(s, 3H), 5.55(s, 1H), 7.56(bs, 2H)

MS(EI, M): 171, 156

Preparation Example 6: Synthesis of 4-amino-6-carboxymethoxy-1-methyl-2-pyrimidinethione

The same procedures as described in Preparation Example 5 above were repeated except that 17.1g of 4-amino-1-methyl-2-methylthiopyrimidin-6-one and 19.5g of t-butyl bromoacetate were used and the reaction nixture was heated under reflux for 72 hours to obtain 9.8g of the title compound.

m.p.: 208 ° C

NMR(δ, DMSO-$d_6$): 3.75(s, 3H), 4.91(s, 2H), 5.57(s, 1H), 7.47(bs, 2H)

Preparation Example 7: Synthesis of 4-amino-6-phenyl-1-methyl-2-pyrimidinethione

To a solution of 1g of 3-methoxy-3-phenyl acrylonitrile and 0.57g of N-methylthiourea dissolved in 5 ml of DMF was added 0.85g of sodium ethoxide. The resultant mixture was heated to reflux for 18 hours, and evaporated under a reduced pressure to remove DMF solvent, and the resulting residues were separated by a column chromatography to obtain 0.49g of the title compound.

m.p.: 217 ° C

NMR(δ, DMSO-$d_6$): 3.89(2, 3H), 6.63(s, 1H), 7.52(m, 3H), 7.94(dd, 2H), 7.96(bs, 2H)

Preparation Example 8: Synthesis of 4-amino-5-phenyl-1-methyl-2-pyrimidinethione

To a solution of 3g of 3-methoxy-2-phenylacrylonitrile dissolved in 15 ml of DMF were added 1.92g of sodium ethoxide and 1.70g of N-methyl thiourea. The reaction mixture was heated under reflux for 6 hours and evaporated under a reduced pressure to remove DMF solvent. To the residues thus obtained was added 10 ml of water. The resultant solution was adjusted to pH 4 and thereto was added 100 ml of ethyl acetate to produce white colored precipitates , which were filtered, washed with 50 ml of ethylacetate and dried to obtain 1.30g of the title compound.

m.p.: 228 ° C

NMR(δ, DMSO-$d_6$): 3.71(s, 3H), 7.36-7.52(m, 7H), 6.9(s,1H)

Preparation Example 9: Synthesis of 4-amino-1-carboxymethyl-2-pyrimidinethione

A solution of 14.1g of 4-amino-2-methylthiopyrimidine and 30g of methyl bromoacetate dissolved in 70 ml of THF was heated under reflux for 10 hours and cooled to produce precipitates, which were filtered and dried. To the dried solids were added 8.5g of NaSH and 60 ml of ethanol, and the resultant mixture was heated under reflux to produce precipitates, which were filtered and dried to obtain 2.8g of the title compound.

m.p.: 178 °C

NMR(δ, DMSO-$d_6$): 7.82(d, 1H), 7.60(d, 2H), 6.02(d, 1H), 4.98(s, 2H)

Preparation Example 10: Synthesis of 4-amino-5-carboxy-1-methyl-2-pyrimidinethione

Step 1) synthesis of 4-amino-2-methylthiopyrimidyl-5-methylcarboxylate

To 50 ml of methanol were added 15.5g of methyl-2-ethoxymethylene cyanoacetate and 9.0g of N-methylthiourea and thereto was added 5.4g of $NaOCH_3$. The resultant mixture was stirred at room temperature for 1 hour and then refluxed for 12 hours, and distilled under a reduced pressure to obtain residues, which were separated by a column chromatography to obtain 4-amino-2-thiopyrimidyl-5-methyl carboxylate as an intermediate. The intermediate thus obtained was treated with methyl iodide to obtain 4.2g of the title compound.

m.p.: 207 °C

NMR(δ, DMSO-$d_6$): 8.08(s, 1H), 8.42(s, 2H), 3.80(s, 3H), 2.56(s, 3H)

Step 2) Synthesis of 4-amino-5-carboxy-1-methyl-2-pyrimidinethione

To 20g of the compound obtained in step 1 were added 20g of methyl iodide and 100 ml of tetrahydrofuran. The reaction mixture was heated under reflux for 20 hours and cooled to produce precipitates, which were filtered and dried. To the solids thus obtained were added 100 ml of ethanol and 10g of NaSH, and the resultant solution was heated under reflux and cooled to produce white-colored precipitates, which were filtered, dried and treated with sodium hydroxide to obtain 3.2g of the title compound.

m.p.: 229 °C

NMR(δ, DMSO-$d_6$): 8.69(s, 1H), 8.32(bs, 1H), 7.78(bs, 1H), 3.72(s, 3H)

Preparation Example 11: Synthesis of 4,5-diamino-1-methyl-2-pyrimidinethione

A mixture of 15.6g of 4,5-diamino-2-methylthio pyrimidine, 20g of methyl iodide and 80 ml of THF was heated under reflux for 36 hours and cooled to produce precipitates, which were filtered, dried and treated with NaSH to obtain 3.6g of the title compound.

m.p.: 208 °C

NMR(δ, DMSO-$d_6$) : 7.91(s, 1H), 7.24(s, 2H), 3.80(s, 3H), 3.58(bs, 2H)

Preparation Example 12: Synthesis of 4-amino-1-methylbenzo[5,6,e]pyrimidine-2-thione

Step 1) Synthesis of N'-(2-cyanophenyl)-N'-methyl-N''-benzoyl thiourea

To a solution of 4.36 g of benzoyl isothiocyanate dissolved in 30 ml of acetone was slowly added a solution of 5.67g of N-methylamino-2-cyanobenzene dissolved in 40ml of acetone with heating. The reaction solution was distilled under a reduced pressure to remove acetone solvent to obtain residues, which were separated by a column chromatography (using as an eluent a mixture of hexane and ethyl acetate (5:2)) to obtain 2.97g of the title compound.

NMR(δ, $CDCl_3$): 8.50(s, 1H), 7.3-7.8(m, 9H), 3.79(s, 3H)

Step 2) Synthesis of 4-amino-1-methylbenzo[5,6,e]pyrimidine-2-thione

A mixture of 2.97g of the compound obtained in step 1, 0.4g of NaOH and 20 ml of ethanol was heated under reflux for 4 hours. After rapid filtration, the filtrate was cooled with an iced water to produce white-colored precipitates, which were filtered and dried to obtain 0.7g of the title compound.

m.p.: 285 ° C

NMR(δ, DMSO-$d_6$): 8.32(s, 2H), 8.12(d, 1H), 7.80(dd, 1H), 7.56(d, 1H), 7.31(dd, 1H), 4.08(s, 3H)

Preparation Example 13: Synthesis of 6-amino-3-methylpyrido [5,6,e]pyrimidine-4-thione

To a solution of 4.8g of NaH dissolved in 10 ml of dry THF was added 0.9g of N-methyl thiourea. To the resulting mixture was slowly added a solution of 1.38g of 2-chloro-3-cyanopyridine dissolved in 10 ml of dry DMF and the resultant was stirred at room temperature for 2 hours. Into 200 ml of an iced water was poured the above resultant solution to produce precipitates, which were filtered, washed three times with 100 ml of water and dried to obtain 1.7g of the title compound.

m.p.: 272 ° C

NMR(δ, DMSO-$d_6$): 8.81(d, 1H), 8.56(d, 1H), 8.4-8.6(bd, 2H), 7.42(dd, 1H), 4.02(s, 3H)

Preparation Example 14: Synthesis of 4-amino-1-methylfuro [5,6,e]pyrimidine-2-thione

Step 1) Synthesis of 4-amino-1-methyl-7-hydroxytetrahydrofuro[5,6,e]pyrimidine-2-thione

To a solution of 1.71g of 4-amino-1-methyl-2-thiomethylpyimidin-6-one dissolved in 10 ml of water were added 0.82g of NaOAc and 9.4g of chloroacetaldehyde. The resultant mixture was heated to 100 ° C and treated with NaSH to obtain 0.28g of the title compound.

m.p.: 198 ° C

NMR(δ, DMSO-$d_6$): 7.88(s, 2H), 5.50(s, 1H), 5.32(d, 1H), 4.69(m, 1H), 4.37(d, 1H), 3.97(s, 3H)

Step 2) Synthesis of 4-amino-1-methylfuro[5,6,e]pyrimidine-2-thione

A mixture of 1.87g of the compound obtained in step 1 and 10 ml of conc. HCl was stirred at a room temperature for 1 hour and cooled to 0 ° C. Thereto was added 100 ml of ethyl ether to obtain 1.43 g of the title compound as a white solid.

m.p.: 298 ° C

NMR(δ, DMSO-$d_6$): 7.89(d, 1H), 7.28(d, 1H), 7.20(bs, 2H), 3.72(s, 3H)

Preparation Example 15: Synthesis of 4-amino-5-benzyl-1-methylpyrimidine-2-thione

Into 100 ml of ethanol were charged 9.7g of ethoxy acrylonitrile, 9.1g of methyl thiourea and 11.0g of benzaldehyde. The resultant mixture was heated under reflux for 24 hours with 12.3 g of NaOEt and cooled to produce precipitates, which were filtered and dried to obtain 4.8g of the title compound.

m.p.: 298 ° C

NMR(δ, DMSO-$d_6$): 7.78(s, 1H), 7.2-7.5(m, 7H), 3.68 (s, 2H), 3.62(s, 3H)

Preparation Example 16: Synthesis of 4-amino-6-methylamino-1-methylpyrimidine-2-thione

A mixture of 1.56g of 4.5-diamino-1-methylpyrimidine-2-thione, 0.23g of NaH and 8 ml of DMF was cooled to 0 ° C and thereto was added 1.48 g of methyl iodide. The reaction solution was warmed slowly to a room temperature and thereto was added 1 ml of water. The resultant was distilled under a reduced pressure to remove the DMF solvent and the residues thus obtained was washed with 20 ml of methanol and 20 ml of water to obtain 0.78g of the title compound.

m.p.: 288 ° C

NMR(δ, DMSO-$d_6$): 7.82(m, 1H), 7.60(bs, 2H), 5.18(s, 1H), 3.72(s, 3H), 2.75(d, 3H)

Preparation Example 17: Synthesis of 4-amino-6-ethylamino-1-methylpyrimidine-2-thione

The same procedures as described in Preparation Example 16 were repeated except that methyl iodine was substituted with ethyl iodide to obtain 0.64 g of the title compound.

m.p.: 295 ° C

NMR(δ, DMSO-$d_6$): 7.92(m, 1H), 7.61(bs, 2H), 5.28(s, 1H), 3.82(q, 3H), 2.85(d, 3H), 1.24(t, 3H)

Preparation Example 18: Synthesis of 4-amino-5-formamido-1-methylpyrimidine-2-thione

Step 1) Synthesis of 4-amino-2-methylthio-5-methoxycarbonyl-1-methylpyrimidinium iodide

A mixture of 4g of the compound obtained in step 1 of Preparation Example 10 above, 15g of methyl iodide and 50 ml of THF was heated under reflux for 18 hours, filtered and dried to obtain 10g of the title compound.

M.p.: 218 °C

NMR(δ, DMSO-$d_6$): 8.68(s, 1H), 8.20(s, 2H), 3.83(s, 3H), 3.78(s, 3H), 2.50(s, 3H)

Step 2) Synthesis of 4-amino-5-formamido-1-methylpyrimidine-2-thione

A mixture of 10g of the compound obtained in step 1, 10g NaSH and 100 ml of ethanol was heated under reflux for 8 hours and filtered to obtain solids, which were washed with water and dried. To the dried solids was added 100 ml of an ammonium hydroxide solution and the resultant solution was heated for 4 hours and acidified to obtain 1.46g of the title compound.

m.p.: 288 °C

NMR(δ, DMSO-$d_6$): 8.70(s, 1H), 8.20(d, 2H), 3.63(s, 3H)

Preparation Example 19: Synthesis of 1,4-diaminopyrimidine-2-thione

Step 1 ) Synthesis of 4-amino-2-methylthiopyrimidine

The same procedures described in Preparation Example 2 above were repeated except for using 97.12g of 3-ethoxy acrylonitrile and 76.07g of thiourea to obtain an intermediate. The intermediate thus obtained was treated with 14g of methyl iodide in the presence of tetrahydrofuran and water as a solvent to obtain the title compound.

Setp 2) Synthesis of 1,4-diaminopyrimidine-2-thione

The same procedures described in Preparation Example 21 were repeated except for using the compound obtained in step 1 to obtain the title compound.

m.p.: 292 °C

NMR(δ, DMSO-$d_6$): 7.90(d, 1H), 7.52(bs, 2H), 6.51(bs, 2H), 6.02(d, 1H)

Preparation Example 20: Synthesis of 1,4,5-triaminopyrimidine-2-thione

The same procedures described in Step 2 of Preparation Example 1 above were repeated except for using 1,4-diaminopyrimidine-2-thione obtained in Preparation Example 19 above to obtain the title compound.

m.p.: 274 °C

NMR(δ, DMSO-$d_6$): 7.94(s, 1H), 6.38(s, 2H), 5.20(bs, 2H), 3.32(s, 2H)

Preparation Example 21: Synthesis of 1,4,5,6-tetraamino pyrimidine-2-thione

To a solution of 17.1g of 4,5,6-triamino-2-methylthio pyrimidine, which was obtained in Preparation Example 1 above, dissolved in 100 ml of dichloromethane was added dropwise a solution of 43g of hydroxylamine-0-mesitylene sulfonate dissolved in 50 ml of dichloromethane at 0 °C. The reaction solution was slowly warmed to a room temperature and stirred for about 10 minutes. Thereto was added 300 ml of ethyl ether to produce solids, which were filtered, washed with 200 ml of ethyl ether and dried to provide a N-aminosalt. A solution of the salt dissolved in 15 ml of methanol was treated with NaSH and subjected to a column chromatography to obtain 5.2g of the title compound.

m.p.: 278 °C

NMR(δ, DMSO-$d_6$): 7.60(s, 2H), 7.42(s, 2H), 6.48(s, 2H), 3.32(s, 2H)

Preparation Example 22: Synthesis of 4-amino-1-methyl-6-(N,N'-dimethyl)aminopyrimidine-2-thione

To a mixture of 17.55g of 4-amino-6-chloro-2-methylthio pyrimidine and 50 ml of ethanol was added 13.5g of N,N-dimethylamine. The resultant mixture was heated under reflux for 24 hours and distilled under a reduced pressure to remove ethanol solvent. Thereto was added a solution of 27.88g of chlorotriphenylmethane dissolved in 75 ml of pyridine. The resultant solution was distilled under a reduced pressure to obtain residues, which were washed with 100 ml of 1N-HCl and 200 ml of ethyl acetate. The organic phase was dried and filtered, and the filtrate was distilled under a reduced pressure to produce solids. A mixture of the obtained solids and methyl iodide was heated under reflux for 20 hours in the presence of tetrahydrofuran to produce solids, which were filtered, dried, subjected to the deprotection procedure and treated with NaSH to obtain 3.8g of the title compound.

M.p.: 207°C

NMR($\delta$, DMSO-$d_6$): 6.98(bs, 2H), 5.26(s, 1H), 3.72(s, 3H), 2.90(s, 6H)

Preparation Example 23: Synthesis of 1,4-diamino-5-methyl-2-pyrimidinethione

The same procedures as described in Preparation Example 21 above were repeated except for using 15.5g of 4-amino-5-methyl-2-thiomethylpyrimidine and 43g of hydroxylamine-0-mesitylene sulfonate to obtain 8.9g of the title compound.

m.p.: 218°C

NMR($\delta$, DMSO-$d_6$): 8.05(s, 1H), 7.57(bs, 2H), 6.20(bs, 2H), 1.82(s, 3H)

Preparation Example 24: Synthesis of 1,4-diamino-5-ethyl-2-pyrimidinethione

The same procedures as described in Preparation Example 23 above were repeated except that 4-amino-5-methyl-2-thiopyrimidine was substituted with 4-amino-5-ethyl-2-thiopyrimidine to obtain the title compound.

m.p.: 228°C

NMR($\delta$, DMSO-$d_6$): 8.06(s, 1H), 7.52(bs, 2H), 6.18(bs, 2H), 1.98(q, 2H), 1.05(t, 3H)

Preparation Example 25: Synthesis of 1,4-diamino-6-(N-methyl) amino-2-pyrimidinethione

To a solution of 17.2g of 1,4,6-triamino-2-methylthio pyrimidine dissolved in 80 ml of DMF was added 5.4g of sodium methoxide and then added dropwise 14.19g of methyl iodide. The materials obtained after filtration of the reaction mixture were treated with NaSH to obtain 7.2g of the title compound.

m.p.: 247°C

NMR($\delta$, DMSO-$d_6$): 7.21(q, 1H), 6.68(bs, 2H), 5.81(s, 2H), 5.02(s, 1H), 2.71(d, 3H)

Preparation Example 26: Synthesis of 1,4-diamino-5-methyl-6-(N-methyl)amino-2-pyrimidinethione

The same procedures as described in Preparation Example 25 above were repeated except for using 1,4,6-triamino-5-methyl-2-methylthiopyrimidine to obtain the title compound.

m.p.: 278°C

NMR($\delta$, DMSO-$d_6$): 6.87(q, 1H), 6.69(bs, 2H), 5.92(s, 2H), 3.02(d, 3H), 1.98(s, 3H)

Preparation Example 27: Synthesis of paramethoxybenzyl 3-chloromethyl-7-{(z)-2-[2-(triphenylmethyl)-aminothiasol-4-yl]-2-[1-(diphenylmethoxycarbonyl)cyclobutoxyimino]acetamido}-3-cephem-4-carboxylate

Step 1) Synthesis of diphenylmethyl 1-bromocyclobutane carboxylate

To 50g of cyclobutane carboxylic acid was added 1g of red phosphorus and the reaction solution was heated at a temperature ranging from 130 to 140°C with stirring while 96g of bromine was added dropwise over 3 hours. The resultant solution was further stirred for 1 hour and cooled to a room temperature, and thereto were added 300 ml of ethyl ether and 300 ml of water. The mixture was stirred for 5 minutes, and the organic phase was dried over magnesium sulfate and filtered. To the dried organic phase was added dropwise, with stirring, 1M solution of diphenyl diazomethane dissolved in ethyl ether until no bubbles were generated, and the resultant solution was washed with 500 ml of 5% sodium bicarbonate aqueous solution and 500 ml of saturated sodium chloride solution. The organic phase was dried over magnesium sulfate,

filtered and concentrated under a reduced pressure to obtain 93g of the title compound in the form of oil.

Step 2) Synthesis of (z)-2-{[2-(triphenylmethyl)aminothiazol-4-yl]-2-[1-(diphenylmethoxycarbonyl)-cyclobutoxyimino]} acetic acid

To a solution of 46.9g of allyl 2-[2-(triphenylmethyl)aminothiazol-4-yl]-2-hydroxyiminoacetate dissolved in 200 ml of dimethyl sulfoxide were added 27.2g of potassium carbonate and 37g of the compound obtained in step 1. The resultant solution was heated to a temperature ranging from 40 to 50 °C, stirred for 8 hours at the temperature range, cooled to a room temperature and thereto were added 1l of ethyl acetate and 1l of distilled water. The resultant was stirred for 5 minutes and the organic phase was separated therefrom, washed twice with 1l of saturated sodium choloride solution, dried over magnesium sulfate, filtered and concentrated under a reduced pressure. The concentrated residues were dissolved in 400 ml of dichloromethane and to the resultant solution were added in order 2.6g of triphenylphosphine, 0.1g of tetrakis(triphenylphosphine)palladium and a solution of 19g of potassium 2-ethylhexanoate dissolved in 200 ml of ethyl acetate. The solution was stirred at a room temperature for 2 hours. To the reaction solution were added 500 ml of ethyl acetate and 1l of saturated sodium chloride solution. The aqueous phase was adjusted to about pH 3 with 2N HCl solution and the organic phase was separated, dried over magnesium sulfate, filtered, and concentrated under a reduced pressure to obtain 27.6g of the title compound in the form of a foam.

NMR($\delta$, $CDCl_3$) : 1.70~2.10(m, 2H), 2.25~2.60(m, 4H), 6.36 (s, 1H), 6.87(s, 1H), 6.95~7.55(m, 26H), 8.90(bs, 1H)

Step 3) Synthesis of paramethoxybenzyl 3-chloromethyl-7-{(z)-2-[2-(triphenylmethyl)aminothizol-4-yl)-2-[1-(diphenyl methoxycarbonyl)cyclobutoxyimino]acetamido}-3-cephem-4-carboxylate

A suspension of 8.5g of paramethoxybenzyl 7-amino-3-chloromethyl-3-cephem-4-carboxylate hydrochloride and 13.7g of the compound obtained in step 2 dispersed into 100 ml of dichloromethane was cooled to -20 °C and thereto were added 5.4g of pyridine and 3.1g of phosphorous oxychloride while maintaining the temperature . The reaction mixture was stirred for 20 minutes and warmed to a room temperature over 1 hour. Thereto were added 200 ml of ethyl acetate and 200 ml of 1% HCl solution, and the resultant solution was stirred for 5 minutes. The organic phase was separated, washed with 200 ml of saturated sodium chloride solution, dried over magnesium sulfate, filtered and concentrated under a reduced pressure to obtain 18.2g of the title compound in the form of a foam.

NMR($\delta$, $CDCl_3$) : 2.00~2.15(m, 2H), 2.45~2.80(m, 4H), 3.15 ~ 3.45(ABq, 2H), 3.79(s, 3H), 4.35 ~ 4.56-(ABq, 2H), 4.91(d, 1H), 5.22(q, 2H), 5.92(q, 1H), 6.68(s, 1H), 6.88(d, 2H), 6.90(s, 1H), 7.08(s, 1H), 7.15~7.40(m, 27H), 7.55(d, 1H)

Preparation Example 28: Synthesis of paramethoxybenzyl 3-chloromethyl-7-{(z)-2-[2-(triphenylmethyl)-aminothiazol-4-yl]-2-[1-(diphenylmethoxycarbonyl)cyclopentoxyimino]acetamido}-3-cephem-4-carboxylate

The same procedures as described in Preparation Example 27 above were repeated except that as a starting material 57g of cyclopentane carboxylic acid was used to obtain 18.1g of the title compound in the form of a foam.

NMR($\delta$, $CDCl_3$) : 1.65~1.86(m, 4H), 2.10~2.25(m, 2H), 2.32 ~2.55(m, 2H), 3.22~3.47(ABq, 2H), 3.80(s, 3H), 4.40~4.55(ABq, 2H), 5.02(d, 1H), 5.23(q, 2H), 5.95(q, 1H), 6.67(s, 1H), 6.88(s, 1H), 6.90 (d, 2H), 7.05~7.42(m, 28H), 7.46(d, 1H)

Preparation Example 29: Synthesis of paramethoxybenzyl 3-chloromethyl-7-{(z)-2-[2-(triphenylmethyl)-aminothiazol-4-yl]-2-[1-(diphenylmethoxycarbonyl)cyclohexoxyimino]acetamido}-3-cephem-4-carboxylate

The same procedures as described in Preparation Example 27 above were repeated except that as a starting material 64g of cyclohexane carboxylic acid was used to obtain 18.3g of the title compound in the form of a foam.

NMR($\delta$, $CDCl_3$) : 1.20~1.80(m, 6H), 1.80~2.10(m, 2H), 2.28 ~2.35(m, 2H), 3.25~3.48(ABq, 2H), 3.75 (s, 3H), 4.36~4.55(ABq, 2H), 4.91 (d, 1H), 5.22(q, 2H), 5.90(q, 1H), 6.62(s, 1H), 6.88 (s, 1H), 6.90(d, 2H), 7.10~7.55(m, 28H), 7.77 (d, 1H)

Preparation Example 30: Synthesis of paramethoxybenzyl 3-chloromethyl-7-{(z)-2-[2-(triphenylmethyl)-aminothiazol-4-yl]-2-[(R)-1-diphenylmethoxycarbonylprop-1-oxyimino]acetamido}-3-cephem-4-carboxylate

Step 1) Synthesis of S-(-)-2-chlorobutanoic acid

A solution of 25.78g of S-(+)-2-aminobutanoic acid dissolved in 400 ml of 6M HCl solution was cooled to 0°C and thereto was added dropwise a solution of 27.6g of sodium nitrite dissolved in 100 ml of distilled water while maintaining below 5°C. The reaction solution was warmed to a room temperature, stood up for 18 hours and stirred for 3 hours with degassing. Thereto was slowly added 25g of sodium bicarbonate, and the reaction mixture was extracted four times with 100 ml of ethyl ether.

The separated organic phase was concentrated under a reduced pressure to remove the solvent and the volatile materials. The residues thus obtained were dissolved in 4 ml of saturated sodium chloride solution and the resultant solution was extracted with 30 ml of ethyl ether. The orgarnic layer was dried over calcium chloride and filtered, and the filtrate was distilled at a temperature ranging from 40 to 50°C under a reduced pressure to obtain 17.43g of the title compound.

NMR($\delta$, $CDCl_3$) : 1.05(t, 3H), 1.88-2.09(m, 2H), 4.27(dd, 1H), 11.35(bs, 1H)

$[\alpha]^{20}_D$ = -13.07(c=6.71, methanol)

Step 2) Synthesis of S-(-)-2-chlorobutanoic benzhydryl ester

16.7g of S-(-)-2-chlorobutanoic acid obtained in step 1 was dissolved in 100 ml of diethyl ether and thereto was added dropwise 1M solution of diphenyldiazo methane dissolved in diethyl ether until no generation of nitrogen gas was observed. After the completion of the reaction, 200 ml of 5% sodium bicarbonate solution was added to the reaction solution. The solution was vigorously mixed, and the organic phase separated therefrom was dried over anhydrous magnesium sulfate and filtered. The filtrate was distilled under a reduced pressure to remove the solvent, and purified by a silica gel column chromatography to obtain 34g of the title compound.

NMR($\delta$, $CDCl_3$): 0.95(t, 3H), 1.87-2.15(m, 2H), 4.30(dd, 1H), 6.90(s, 1H), 7.20-7.40(m, 10H)

$[\alpha]^{20}_D$ = -6.03(c=3.17, methanol)

Step 3) Synthesis of allyl 2-(tritylaminothiazol)-4-yl]-2-[(R)-(+)-1-(diphenylmethoxycarbonyl)prop-1-oxyimino] acetate

55.91g of allyl[2-(tritylamino)thiazol-4-yl]-2-hydroxyiminoacetate and 34 g of the compound obtained in step 2 were dissolved in 200 ml of dimethyl sulfoxide and thereto was added 32.1g of potassium carbonate. The resultant solution was stirred for 1.5 hours at 55°C. After completion of the reaction, to the solution was added 500ml of distilled water and the resultant solution was extracted with 500ml of diethyl ether. The orgainic phase was separated, washed with 100 ml of 10% HCl solution and 100 ml of saturated sodium chloride solution, dried over anhydrous magnesium sulfate, filtered, concentrated under a reduced pressure to obtain 75.58g of the title compound.

NMR($\delta$, $CDCl_3$) : 0.92(t, 3H), 1.80~2.00(m, 2H), 4.79(d, 2H), 4.88(dd, 1H), 5.20(d, 1H), 5.38(d,1H), 5.80~6.00(m, 1H), 6.50(s, 1H), 6.90(s, 1H), 7.17~ 7.38(m, 26H)

$[\alpha]^{20}_D$ = +9.87°(c=14.35, ethyl acetate)

Step 4) Synthesis of 2-(tritylaminothiazol-4-yl)-2-[(R)-(+)-1-(diphenylmethoxycarbonyl)-prop-1-oxyimino]-acetic acid

To a solution of 75.58g of the compound obtained in Step 3 dissolved in 500ml of dichloromethane were added in order 2.75g of triphenylphosphine, 0.61g of tetrakis(triphenylphosphine)palladium and a solution of 20.1g of potassium 2-ethylhexanoate dissolved in 120ml of ethyl acetate. The resulting mixture were stirred at room temperature for 1 hour. After completion of the reaction, the solution was distilled under a reduced pressure to remove the solvent. To the residues were added 500ml of ethyl acetate and 400ml of distilled water. The resultant solution was adjusted to pH 2.4 with 10% HCl solution and filtered to collect the solids, which were washed with 500 ml of distilled water and filtered to obtain 64.02g of the title compound.

NMR($\delta$, DMSO-$d_6$) : 0.83(t, 3H), 1.65 ~ 1.90 (m, 2H), 4.68(dd, 1H), 6.80(s, 1H), 6.83(s, 1H), 7.10~7.50-(m, 25H), 8.85(s, 1H), 13.80(bs, 1H)

$[\alpha]^{20}_D$ = +1.36°(c=8.9, dimethyl sulfoxide)

Step 5) Synthesis of paramethoxybenzyl 3-chloromethyl-7{(Z)-2-[2-(triphenylmethyl)aminothiazol-4-yl]-2-[-(R)-1-diphenylmethoxycarbonylprop-1-oxyimino]acetamido}-3-cephem-4-carboxylate

Into 1l of dichloromethane were charged 86.68g of paramethoxybenzyl 7-amino-3-chloromethyl-3-cephem-4-carboxylate and 50.64g of pyridine. The mixture was stirred until it became a complete solution and the solution was cooled to a temperature ranging from -20 ° C to -25 ° C and thereto were added 135.5g of the compound obtained in step 4 and 32.8g of phosphorous oxychloride. The reaction mixture was stirred for 30 minutes. After completion of the reaction, the mixture was washed with 500ml of 1% HCl solution. The organic phase was separated therefrom, washed with 500ml of distilled water and with 500ml of saturated sodium chloride solution, dried over anhydrous magnesium sulfate and filtered. The filtrate was concentrated under a reduced pressure to obtain 185g of the title compound as an ivory solid.

NMR(δ, $CDCl_3$) : 1.06(t, 3H), 1.95(q, 2H), 3.29(ABq, 2H), 3.81 (s, 3H), 3.97(ABq, 2H), 4.95(d, 1H), 5.02-(dd, 1H), 5.21(ABq, 2H), 5.92(dd, 1H), 7.1(ABq, 4H), 6.72(s, 1H), 6.95(s, 1H), 8.08(d, 1H)

Preparation Example 31: Synthesis of paramethoxybenzyl 3-chloromethyl-7-{(Z)-2-[2-(triphenylmethyl)-aminothiazol-4-yl]-2-[(R,S)-1-diphenylmethoxycarbonylprop-1-oxyimino]acetamido}-3-cephem-4-carboxylate

The same procedures described in Steps 2, 3, 4 and 5 of Preparation Example 30 above were repeated except that 17.4g of (±)-2-bromobutanoic acid was used as a starting material to obtain 185g of the title compound as a white solid.

Preparation Example 32: Synthesis of paramethoxybenzyl 3-chloromethyl-7{(Z)-2-[2-(triphenylmethyl)-aminothiazol-4-yl]-2-[(R,S)-2-diphenylmethoxycarbonylbut-1-oxyimino]acetamido}-3-cephem-4-carboxylate

Step 1) Synthsis of allyl 2-(tritylaminothiazol-4-yl)-2-[2-(diphenylmethoxycarbonyl)but-2-oxyimino]acetate

To a solution of 250g of allyl [2-(tritylamino)thiasol-4-yl]-2-hydroxyiminoacetate and 200g of 2-bromo-2-methylbutanoic benzhydryl ester dissolved in 600ml of dimethyl sulfoxide was added 150g of potassium carbonate. The resultant solution was stirred at 60 ° C for 18 hours, and the solution thoroughly was poured into a mixture of 3l of ethyl acetate and 1.5l of an iced water. The resultant was stirred thoroughly and the organic phase was separated therefrom, washed with 1 ℓ of 1% HCl solution and then with 1 ℓ of saturated sodium chloride solution, dried over magnesium sulfate and filtered. The filtrate was concentrated under a reduced pressure to obtain 350g of the title compound as a white solid.

NMR(δ, $CDCl_3$) : 0.81(t, 3H), 1.54(s, 3H), 1.91(q, 2H), 4.82 (d, 2H), 5.31(ABq, 2H), 5.86~6.02(m, 1H), 6.47(s, 1H), 6.74(s, 1H), 6.86(s, 1H), 7.12~ 7.41(m, 25H)

Step 2) Synthesis of 2-(tritylaminothiazol-4-yl)-2-[2-(diphenylmethoxycarbonyl)-but-2-oxyimino]acetic acid

350g of the compound obtained in Step 1 above was dissolved in 1.4l of dichloromethane and thereto were added in order 17.2g of triphenylphosphine, 3.05g of tetrakis (triphenylphosphine)palladium and a solution of 95.25g of potassium 2-ethylhexanoate dissolved in 600ml of ethyl acetate. The solution was stirred at a room temperature for 4 hours. The solution was distilled under a reduced pressure to obtain residues to which were added a mixture of 600ml of ethyl acetate and 600ml of distilled water. The resultant solution was adjusted to about pH 2.8 with 5% HCl solution and the organic phase was separated therefrom, dried over anhydrous magnesium sulfate and filtered. The filtrate was distilled under a reduced pressure to obtain residues, which were purified by a silicagel column choamatography to obtain 280g of the title compound as a white solid.

NMR(δ, $CDCl_3$) : 0.72(t, 3H), 1.57(s, 3H), 1.87(q, 2H), 6.33 (s, 1H), 6.90(s, 1H), 7.14~7.42(m, 26H), 9.02 (bs, 1H)

Step 3) Synthesis of paramethoxylbenzyl 3-chloromethyl-7{(Z)-2-[2-(triphenylmethyl)aminothiazol-4-yl]-2-[-(R,S)-2-diphenylmethoxycarbonylbut-1-oxyimino]acetamido}-3-cephem-4-carboxylate

Into 1l of dichloromethane were charged 86.7g of parmethoxybenzyl 7-amino-3-chloromethyl-3-cephem-4-carboxylate and 50.64g of pyridine. After the mixture was stirred until it became a complete solution, the solution was cooled to a temperature ranging from -20 to -25 ° C and 135.5g of the compound obtained in Step 2 and 32.8g of phosphorous oxychloride were added thereto. The reaction mixture was stirred for 30 minutes. After completion of the reaction, the mixture was washed with 500ml of 1% HCl solution and the

organic phase was separated therefrom, washed with 500ml of distilled water and then 500ml of saturated sodium chloride solution, dried over anhydrous magnesium sulfate and filtered. The filtrate was concentrated under a reduced pressure to obtain 185g of the title compound as an ivory solid.

NMR(δ, $CDCl_3$) : 0.85~0.89(m, 3H), 1.69(d, 3H), 1.87~2.24 (m, 2H), 3.08~3.17(m, 2H), 3.85(s, 3H), 4.44~4.61(m, 2H), 5.02(dd, 1H), 5.20~5.36 (m, 2H), 5.89~6.01(m, 1H), 6.67(d, 1H), 6.91 (d, 1H), 6.97(d, 1H), 7.15~7.45(m, 29H)

Example 1: Synthesis of 7-[(Z)-2-(aminothiazol-4-yl)-2-(carboxyprop-2-oxyimino)acetamido]-3-(4,5,6-triamino-1-methylpyrimidinium-2-yl)thiomethyl-3-cephem-4-carboxylate

To a solution of 0.92g of paramethoxybenzyl 3-chloromethyl-7-[(Z)-2-(2-tert-butoxycarbonylprop-2-oxyimino)-2-{2-(triphenylmethyl)aminothiazol-4-yl}acetamido]-3-cephem-4-carboxylate dissolved in 4 ml of DMF was added 0.17g of 4,5,6-triaminol-1-methyl-2-pyrimidinethione synthesized in Preparation Example 1 above and the reaction solution was stirred at a room temperature for 1 hour and distilled under reduced pressure to remove the DMF solvent. The residues so obtained was dissolved in 15ml of $CH_2Cl_2$ and the resultant solution was added slowly to 100ml of ethyl ether to produce white precipitates, which were collected by filtration, dried and dissolved in a mixed solvent of 5ml of anisole and 5ml of trifluoroactic acid at -30 °C. The resulting solution was stirred at a room temperature for 1.5 hours and thereto was added 40 ml of ethyl ether to produce white precipitates, which were filtered, washed with 20 ml of acetone and 20 ml of ethyl ether and dried completely to obtain 0.45g of the title compound.

NMR(δ, $D_2O$): 1.58(s, 6H), 3.64(s, 3H), 3.65(ABq, 2H), 4.26(ABq, 2H), 5.19(d, 1H), 5.82(d, 1H), 7.02(s, 1H)

IR(KBr, $cm^{-1}$) : 1772, 1635

MS(FAB, M + 1) : 639

Example 2:Synthesis of 7-[(Z)-2-(aminothiazol-4-yl)-2-(carboxyprop-2-oxyimino)acetamido]-3-(4-amino-1-methylpyrimidinium-2-yl)thiomethyl-3-cephem-4-carboxylate

The same procedures as described in Example 1 were repeated except that 4-amino-1-methyl-2-pyrimidinethione synthesized in Preparation Example 2 above was used as a starting material to obtain 0.39g of the title compound.

NMR(δ, $D_2O/NaHCO_3$): 1.48(s, 6H), 3.58(ABq, 2H), 3.65(s, 3H), 4,40(ABq, 2H), 5.20(d, 1H), 5.82(d, 1H), 6.52(d, 1H), 6.99(s, 1H), 7.85(d, 1H)

IR(KBr, $cm^{-1}$) : 1771, 1630

MS(FAB, M + 1) : 609

Example 3: Synthesis of 7-[(Z)-2-(aminothiazol-4-yl)-2-(ethoxyimino)acetamido]-3-(4,5,6-triamino-1-methylpyrimidinium-2-yl)thiomethyl-3-cephem-4-carboxylate

To a solution of 0.81g of paramethoxybenzyl 3-chloromethyl-7-[(Z)-2-{(2-triphenylmethyl)aminothiazol-4-yl}-2-(ethoxyimino)acetamido]-3-cephem-4-carboxylate dissolved in 4 ml of DMF was added 0.17g of 4,5,6-triamino-1-methyl-2-pyrimidinethione synthesized in Preparation Example 1 above and the reaction solution was stirred at a room temperature for 1 hour and distilled under a reduced pressure to remove the DMF solvent. The residues so obtained was dissolved in 15ml of $CH_2Cl_2$ and the resultant solution was added slowly to 100ml of ethyl ether to produce white pricipitates, which were collected by filtration, dried and dissolved in a mixed solvent of 5ml of anisole and 5ml of trifuoracetic acid at -30 °C. The resulting solution was stirred at room temperature for 30 minutes and thereto was added 40 ml of ethyl ether to produce white precipitates, which were filtered, washed with 20 ml of acetone and 20 ml of ethyl ether and dried completely to obtain 0.35g of the title compound.

NMR(δ, DMSO-$d_6$) : 1.30(t, 3H), 3.58(s, 3H), 3.40(ABq, 2H), 4.20(ABq, 2H), 4.25(q, 2H), 5.20(d, 1H), 5.82(m, 1H), 6.91(s, 1H), 7.2~7.8(m, 4H), 9.72(d, 1H)

IR(KBr, $cm^{-1}$) : 1774, 1631

MS(FAB, M + 1) : 581

Example 4:Synthesis of 7-[(Z)-2-(aminothiazol-4-yl)-2-(ethoxyimino)acetamido]-3-(4-amino-1-methyl pyrimidinium-2-yl)thiomethyl-3-cephem-4-carboxylate

The same procedures as described in Example 3 above were repeated except that 4-amino-1-methyl-2-pyrimidinethione synthesized in Preparation Example 2 above was used as a starting material to obtain 0.40g of the title compound.

NMR(δ, DMSO-$d_6$) : 1.21(t, 3H), 3.40(ABq, 2H), 3.62(s, 3H), 4.05(q, 2H), 4.35(ABq, 2H), 5.02(d, 1H), 5.62(dd, 1H), 6.65(d, 1H), 6.72(s, 1H), 7.24(bs, 2H), 8.12(d, 1H), 9.25(bd, 2H), 9.58(d, 1H)

IR(KBr, $cm^{-1}$) : 1774, 1633

MS(FAB, M + 1) : 551

Example 5:Synthesis of 2-(5-amino-1,2,4-thiadiazol-3-yl)-2-(ethoxyimino)acetamido]-3-(4,5,6-triamino-1-methylpyrimidinium-2-yl)thiomethyl-3-cephem-4-carboxylate

The same procedures as described in Example 3 were repeated except that paramethoxybenzyl 3-chloromthyl-7-[Z]-2-(5-amino-1,2,4-thiadiazol-3-yl)-2-(ethoxyimino)acetamido]-3-cephem-4-carboxylate was used as a starting material to obtain 0.41g of the title compound.

NMR(δ, $D_2O$ + acetone-$d_6$): 1.40(t, 3H), 3.64(s, 3H), 3.45(ABq, 2H), 4.40(ABq, 2H), 4.42(q, 2H), 5.18(d, 1H), 5.80(d, 1H)

IR(KBr, $cm^{-1}$) : 1772, 1630

MS(FAB, M + 1) : 582

Examples 6 to 82

Similary to the procedures described in the above Example 1 to 5, the following compounds were synthesized.

Ex. 6: Synthesis of 7-[(Z)-2-(5-amino-1,2,4-thiadiazol-3-yl)-2-(ethoxyimino)acetamido]-3-(4-amino-1-methyl-pyrimidinium-2-yl)thiomethyl-3-cephem-4-carboxylate

NMR(δ, $D_2O$ + acetone): 1.38(t, 3H), 3.50(s, 3H), 3.42(ABq, 2H), 4.28(ABq, 2H), 4.38(q, 2H), 5.19(d, 1H), 5.78(d, 1H), 6.02(d, 1H), 7.92(d, 1H)

IR(KBr, $cm^{-1}$) : 1772, 1632

MS(FAB, M + 1) : 552

Ex. 7: Synthesis of 7-[(Z)-2-(2-aminothiazol-4-yl)-2-(methoxyimino)acetamido]-3-(4,5,6-triamino-1-methyl-pyrimidinium-2-yl)thiomethyl-3-cephem-4-carboxylate

NMR(δ, $D_2O$ + acetone-$d_6$): 3.60(s, 3H), 3.48(ABq, 2H), 4.45(ABq, 2H), 3.98(s, 3H), 5.20(d, 1H), 5.82(d, 1H), 7.01(s, 1H)

IR(KBr, $cm^{-1}$) : 1770, 1631

MS(FAB, M + 1) : 567

Ex. 8: Synthesis of 7-[(Z)-2-(2-aminothiazol-4-yl)-2-(methoxyimino)acetamido]-3-(4-amino-1-methyl-pyrimidinium-2-yl)thiomethyl-3-cephem-4-carboxylate

NMR(δ, $D_2O$ + acetone-$d_6$): 3.60(s, 3H), 3.50(ABq, 2H), 4.42(ABq, 2H), 4.00(s, 3H), 5.18(d, 1H), 5.80(d, 1H), 6.42(d, 1H), 7.00(s, 1H), 7.98(d, 1H)

IR(KBr, $cm^{-1}$) : 1772, 1630

MS(FAB, M + 1) : 537

Ex. 9: Synthesis of 7-[(Z)-2-(2-aminothiazol-4-yl)-2-oxyiminoacetamido]-3-(4,5,6-triamino-1-methyl-pyrimidinium-2-yl)thiomethyl-3-cephem-4-carboxylate

NMR(δ, $D_2O$+ acetone-$d_6$): 3.59(s, 3H), 3.52(ABq, 2H), 4.40(ABq, 2H), 5.12(d, 1H), 5.81(d, 1H), 6.98(s, 1H)

IR(KBr, $cm^{-1}$) : 1775, 1632

MS(FAB, M + 1) : 559

Ex. 10: Synthesis of 7-[(Z)-2-(2-aminothiazol-4-yl)-2-oxyiminoacetamido]-3-(4-amino-1-methylpyrimidinium-2-yl)thiomethyl-3-cephem-4-carboxylate

NMR(δ, $D_2O$ + aceone-$d_6$): 3.70(s, 3H), 3.54(ABq, 2H), 4.32(ABq, 2H), 5.21(d, 1H), 5.88(d, 1H), 6.50(d, 1H), 7.90(d, 1H)
IR(KBr, $cm^{-1}$) : 1770, 1630
MS(FAB, M + 1) : 529

Ex. 11: Synthesis of 7-[(Z)-2-(2-aminothiazol-4-yl)-2-(carboxyprop-2-oxyimino)acetamido]-3-(4,5,6-triamino-1-ethylpyrimidinium-2-yl)thiomethyl-3-cephem-4-carboxylate

NMR(δ, $D_2O$ + $NaHCO_3$) : 1.20(t, 3H), 1.50(s, 6H), 3.42(ABq, 2H), 4.40(ABq, 2H), 3.88(q, 2H), 5.20(d, 1H), 5.81(d, 1H), 7.01(s, 1H)
IR(KBr, $cm^{-1}$) : 1772, 1632
MS(FAB, M + 1) : 653

Ex. 12: Synthesis of 7-[(Z)-2-(2-aminothiazol-4-yl)-2-(carboxyprop-2-oxyimino)acetamido]-3-(4-amino-1-ethylpyrimidinium-2-yl)thiomethyl-3-cephem-4-carboxylate
NMR(δ, $D_2O$) : 1.18(t, 3H), 1.51(s, 6H), 3.38(ABq, 2H), 3.80(q, 2H), 4.28(ABq, 2H), 5.18(d, 1H), 5.79(d, 1H), 6.51(d, 1H), 7.88(d, 1H)
IR(KBr, $cm^{-1}$) : 1775, 1635
MS(FAB, M + 1) : 623

Ex. 13: Synthesis of 7-[(Z)-2-(2-aminothiazol-4-yl)-2-(ethoxyimino)acetamido]-3-(4,5,6-triamino-1-ethylpyrimidinium-2-yl)thiomethyl-3-cephem-4-carboxylate

NMR(δ, $D_2O$ + acetone): 1.16(t, 3H), 1.40(t, 3H), 3.60(q, 2H), 3.42(ABq, 2H), 4.24(ABq, 2H), 4.20(q, 1H), 5.80(d, 1H), 6.98(s, 1H)
IR(KBr, $cm^{-1}$) : 1772, 1630
MS(FAB, M + 1) : 595

Ex. 14: Synthesis of 7-[(Z)-2-(2-aminothiazol-4-yl)-2-(ethoxyimino)acetamido]-3-(4-amino-1-ethylpyrimidinium-2-yl)thiomethyl-3-cephem-4-carboxylate

NMR(δ, $D_2O$ + acetone-$d_6$): 1.20(t, 3H), 1.50(t, 3H), 3.54(q, 2H), 3.40(ABq, 2H), 4.20(q, 2H), 4.30(ABq, 2H), 5.21(d, 1H), 5.79(d, 1H), 6.54(d, 1H), 7.01(s, 1H), 7.90(d, 1H)
IR(KBr, $cm^{-1}$) : 1774, 1630
MS(FAB, M + 1) : 565

Ex. 15: Synthesis of 7-[(Z)-2-(5-amino-1,2,4-thiadiazol-3-yl)-2-(ethoxyimino)acetamido]-3-(4,5,6-triamino-1-ethylpyrimidinium-2-yl)thiomethyl-3-cephem-4-carboxylate

NMR(δ, $D_2O$ + acetone-$d_6$): 1.18(t, 3H), 1.41(t, 3H), 3.60(q, 2H), 3.42(ABq, 2H), 4.40(ABq, 2H), 4.50(q, 2H), 5.17(d, 1H), 5.78(d, 1H)
IR(KBr, $cm^{-1}$) : 1774, 1632
MS(FAB, M + 1) : 596

EX. 16: Synthesis of 7-[(Z)-2-(5-amino-1,2,4-thiadiazol-3-yl)-2-(ethoxyimino)acetamido]-3-(4-amino-1-ethylpyrimidinium-2-yl)thiomethyl-3-cephem-4-carboxylate

NMR(δ, $D_2O$ + acetone-$d_6$): 1.16(t, 3H), 1.50(t, 3H), 3.58(q, 2H), 3.44(ABq, 2H), 4.42(ABq, 2H), 4.48(q, 2H), 5.20(d, 1H), 5.81(d, 1H), 6.42(d, 1H), 7.82(d, 1H)
IR(KBr, $cm^{-1}$) : 1776, 1632
MS(FAB, M + 1) : 566

Ex. 17: Synthesis of 7-[(Z)-2-(2-aminothiazol-4-yl)-2-(carboxyprop-2-oxyimino)acetamido]-3-(4-amino-6-methoxy-1-methylpyrimidinium-2-yl)thiomethyl-3-cephem-4-carboxylate

NMR(δ, $D_2O$ + acetone-$d_6$): 1.47(s, 6H), 3.57(ABq, 2H), 3.63(s, 3H), 3.97(s, 3H), 4.35(ABq, 2H), 5.21(d, 1H), 5.79(d, 1H), 6.01(s, 1H), 6.93(s, 1H)
IR(KBr, $cm^{-1}$) : 1774, 1632
MS(FAB, M + 1) : 639

Ex. 18: Synthesis of 7-[(Z)-2-(2-aminothiazol-4-yl)-2-(ethoxyimino)acetamido]-3-(4-amino-6-methoxy-1-methylpyrimidinium-2-yl)thiomethyl-3-cephem-4-carboxylate

NMR(δ, $D_2O$) : 1.38(t, 3H), 3.63(ABq, 2H), 3.72(s, 3H), 4.09(s, 3H), 4.31(q, 2H), 4.52(ABq, 2H), 5.27(d, 1H), 5.91(d, 1H), 6.09(s, 1H), 6.98(s, 1H)
IR(KBr, $cm^{-1}$) : 1772, 1630
MS(FAB, M + 1) : 581

Ex. 19: Synthesis of 7-[(Z)-2-(2-aminothiazol-4-yl)-2-(ethoxyimino)acetamido]-3-(4-amino-6-carboxymethoxy-1-methylpyrimidinium-2-yl)thiomethyl-3-cephem-4-carboxylate

NMR(δ, $D_2O/NaHCO_3$) : 1.31(t, 3H), 3.60(ABq, 2H), 3.63(s, 3H), 4.25(q, 2H), 4.29(ABq, 2H), 5.23(d, 1H), 5.81(d, 1H), 6.07(s, 1H), 6.97(s, 1H)
IR(KBr, $cm^{-1}$) : 1776, 1635
MS(FAB, M + 1) : 625

Ex. 20: Synthesis of 7-[(Z)-2-(2-aminothiazol-4-yl)-2-(carboxyprop-2-oxyimino)acetamido]-3-(4-amino-6-phenyl-1-methylpyrimidinium-2-yl)thiomethyl-3-cephem-4-carboxylate

NMR(δ, $D_2O/NaHCO_3$) : 1.48(s, 6H), 3.48(ABq, 2H), 3.76(s, 3H), 4.21(ABq, 2H), 5.21(d, 1H), 5.73(d, 1H), 6.72(s, 1H), 6.93(s, 1H), 7.32(m, 3H), 7.84(d, 2H)
IR(KBr, $cm^{-1}$) : 1775, 1632
MS(FAB, M + 1) : 685

Ex. 21: Synthesis of 7-[(Z)-2-(2-aminothiazol-4-yl)-2-(carboxyprop-2-oxyimino)acetamido]-3-(4-amino-5-phenyl-1-methylpyrimidinium-2-yl)thiomethyl-3-cephem-4-carboxylate

NMR(δ, $D_2O/NaHCO_3$) : 1.52(s, 6H), 3.57(ABq, 2H), 3.73(s, 3H), 4.41(ABq, 2H), 5.18(d, 1H), 5.74(d, 1H), 6.91(s, 1H), 7.46(d, 2H), 7.58(m, 3H), 7.96(s, 1H)
IR(KBr, $cm^{-1}$) : 1774, 1630
MS(FAB, M + 1) : 685

Ex. 22: Synthesis of 7-[(Z)-2-(2-aminothiazol-4-yl)-2-(methoxyimino)acetamido]-3-(4-amino-1-carboxymethylpyrimidinium-2-yl)thiomethyl-3-cephem-4-carboxylate

NMR(δ, DMSO-$d_6$) : 9.53(d, 1H), 9,30, 8.90(s,s. 2H), 7.95(d, 1H), 6.72(s, 2H), 6.46(d, 1H), 5.58(m, 1H), 4.95(d, 1H), 4.28(s, 2H), 4.20 (ABq, 2H), 3.31(ABq, 2H), 3.82(s, 3H)
IR(KBr, $cm^{-1}$) : 1775, 1632
MS(FAB, M + 1) : 579

Ex. 23: Synthesis of 7-[(Z)-2-(2-aminothiazol-4-yl)-2-(ethoxyimino)acetamido]-3-(4-amino-1-carboxymethylpyrimidinium-2-yl)thiomethyl-3-cephem-4-carboxylate

NMR(δ, DMSO-$d_6$) : 9.54(d, 1H), 9.30, 8.91(s,s, 2H), 7.94(d, 1H), 6.73(s, 1H), 6.45(d, 1H), 5.59(m, 1H), 5.00(d, 1H), 4.30(s, 2H), 4.21 (ABq, 2H), 4.05(q, 2H), 3.38(ABq, 2H), 1.45(t, 3H)
IR(KBr, $cm^{-1}$) : 1775, 1632
MS(FAB, M + 1) : 593

Ex. 24: Synthesis of 7-[(Z)-2-(2-aminothiazol-4-yl)-2-(carboxyprop-2-oxyimino)acetamido]-3-(4-amino-1-carboxymethylpyrimidinium-2-yl)thiomethyl-3-cephem-4-carboxylate

NMR($\delta$, $D_2O$) : 7.98(d, 1H), 7.00(d, 1H), 6.49(d, 1H), 5.78(d, 1H), 5.12(d, 1H), 4.32(s, 2H), 4.30(ABq, 2H), 3.30(ABq, 2H), 1.50(s, 6H)
IR(KBr, $cm^{-1}$) : 1775, 1632
MS(FAB, M + 1) : 651

Ex. 25: Synthesis of 7-[(Z)-2-(2-aminothiazol-4-yl)-2-(ethoxyimino)acetamido]-3-(4-amino-5-carboxy-1-methylpyrimidinium-2-yl)thiomethyl-3-cephem-4-carboxylate

NMR($\delta$, DMSO-$d_6$) : 8.21(s, 1H), 6.75(s, 1H), 5.60(m, 1H), 4.98(d, 1H), 4.25(ABq, 2H), 4.09(q, 2H), 3.58-(s, 3H), 3.34(ABq, 2H), 1.25(t, 3H)
IR(KBr, $cm^{-1}$) : 1775, 1635
MS(FAB, M + 1) : 595

Ex. 26: Synthesis of 7-[(Z)-2-(2-aminothiazol-4-yl)-2-(ethoxyimino)acetamido]-3-(4,5-diamino-1-methylpyrimidinium-2-yl)thiomethyl-3-cephem-4-carboxylate

NMR($\delta$, $D_2O$ + acetone-$d_6$): 7.58(s, 1H), 7.01(s, 1H), 5.78(d, 1H), 5.14(d, 1H), 4.40(ABq, 2H), 4.10(q, 2H), 3.54(s, 3H), 3.32(ABq, 2H), 1.24(t, 3H)
IR(KBr, $cm^{-1}$) : 1775, 1635
MS(FAB, M + 1) : 566

EX. 27: Synthesis of 7-[(Z)-2-(2-aminothiazol-4-yl)-2-(carboxyprop-2-oxyimino)acetamido]-3-(4,5-diamino-1-methylpyrimidinium-2-yl)thiomethyl-3-cephem-4-carboxylate

NMR($\delta$, $D_2O$) : 7.57(s, 1H), 7.00(s, 1H), 5.81(d, 1H), 5.18(d, 1H), 4.42(ABq, 2H), 4.11(q, 2H), 3.30(ABq, 2H), 1.50(s, 6H)
IR(KBr, $cm^{-1}$) : 1774, 1632
MS(FAB, M + 1) : 624

Es. 28: Synthesis of 7-[(Z)-2-(2-aminothiazol-4-yl)-2-(carboxyprop-2-oxyimino)acetamido]-3-(4-amino-1-methylbenzo[5,6,e]pyrimidinium-2-yl)thiomethyl-3-cephem-4-carboxylate

NMR($\delta$, $D_2O$) : 7.6~8.1(m, 4H), 6.91(s, 1H), 5.79(d, 1H), 5.16(d, 1H), 4.40(ABq, 2H), 3.81(s, 3H), 3.67-(ABq, 2H), 1.49(s, 6H)
IR(KBr, $cm^{-1}$) : 1775, 1632
MS(FAB, M + 1) : 659

Ex. 29: Synthesis of 7-[(Z)-2-(2-aminothiazol-4-yl)-2-(ethoxyimino)acetamido]-3-(4-amino-1-methylbenzo-[5,6,e]pyrimidinium-2-yl)thiomethyl-3-cephem-4-carboxylate

NNR($\delta$, $D_2O$ + acetone-$d_6$): 8.39(d, 1H), 8.21(m, 1H), 8.05(d, 1H), 7.88(m, 1H), 7.01(s, 1H), 5.91(d, 1H), 5.21(d, 1H), 4.70(ABq, 2H), 4.32(q, 2H), 4.08(s, 3H), 3.81(ABq, 2H), 1.43(t, 3H)
IR(KBr, $cm^{-1}$) : 1774, 1633
MS(FAB, M + 1) : 601

Ex. 30: Synthesis of 7-[(Z)-2-(2-aminothiazol-4-yl)-2-(carboxyprop-2-oxyimino)acetamido]-3-(6-amino-3-methylpyrido[5,6,e]pyrimidinium-4-yl)thiomethyl-3-cephem-4-carboxylate

NMR($\delta$, $D_2O$) : 8.95(d, 1H), 8.60(d, 1H), 7.68(m, 1H), 6.89(s, 1H), 5.79(d, 1H), 5.15(d, 1H), 4.50(ABq, 2H), 3.89(s, 3H), 3.62(ABq, 2H), 1.44(s, 6H)
IR(KBr, $cm^{-1}$) : 1775, 1632
MS(FAB, M + 1) : 660

Ex. 31: Synthesis of 7-[(Z)-2-(2-aminothiazol-4-yl)-2-(ethoxyimino)acetamido]-3-(6-amino-3-methylpyrido-[5,6,e]pyrimidinium-4-yl)thiomethyl-3-cephem-4-carboxylate

NMR($\delta$, DMSO-$d_6$) : 9.50(d, 1H), 9.02(d, 1H), 8.86(d, 1H), 7.70(m, 1H), 7.24(s, 2H), 6.71(s, 1H), 5.60(m, 1H), 4.95(d, 1H), 4.58(ABq, 2H), 4.10(q, 2H), 3.89(s, 3H), 3.31(ABq, 2H), 1.22(t, 3H)
IR(KBr, $cm^{-1}$) : 1772, 1630
MS(FAB, M + 1) : 602

Ex. 32: Synthesis of 7-[(Z)-2-(2-aminothiazol-4-yl)-2-(carboxyprop-2-oxyimino)acetamido]-3-(4-amino-1-methylfuro[5,6,e]pyrimidinium-2-yl-)thiomethyl-3-cephem-4-carboxylate

NMR($\delta$, $D_2O$) : 7.71(d, 1H), 7.02(d, 1H), 6.88(s, 1H), 5.80(d, 1H), 5.21(d, 2H), 4.38(ABq, 2H), 3.62(s, 3H), 3.60(ABq, 2H), 1.51(s, 6H)
IR(KBr, $cm^{-1}$) : 1772, 1632
MS(FAB, M + 1) : 649

Ex. 33: Synthesis of 7-[(Z)-2-(2-aminothiazol-4-yl)-2-(ethoxyimino)acetamido]-3-(4-amino-1-methylfuro-[5,6,e]pyrimidinium-2-yl)thiomethyl-3-cephem-4-carboxylate
NMR($\delta$, DMSO-$d_6$) : 7.81(d, 1H), 7.18(d, 1H), 6.80(s, 1H), 5.70(m, 1H), 5.02(d, 1H), 4.38(ABq, 2H), 4.15-(q, 2H), 3.68(s, 3H), 3.60(ABq, 2H), 1.22(t, 3H)
IR(KBr, $cm^{-1}$) : 1770, 1638
MS(FAB, M + 1) : 501

Ex. 34: synthesis of 7-[(Z)-2-(2-aminothiazol-4-yl)-2-(carboxyprop-2-oxyimino)acetamido]-3-(4-amino-5-benzyl-1-methylpyrimidinium-2-yl)thiomethyl-3-cephem-4-carboxylate

NMR($\delta$, $D_2O$) : 7.52(s, 1H), 7.38(m, 5H), 5.81(d, 1H), 5.12(d, 1H), 4.42(ABq, 2H), 3.82(s, 2H), 3.52(s, 3H), 1.49(s, 6H)
IR(KBr, $cm^{-1}$) : 1774, 1632
MS(FAB, M + 1) : 699

Ex. 35: Synthesis of 7-[(Z)-2-(2-aminothiazol-4-yl)-2-(carboxyprop-2-oxyimino)acetamido]-3-(4-amino-6-methylamino-1-methylpyrimidinium-2-yl)thiomethyl-3-cephem-4-carboxylate

NMR($\delta$, $D_2O$) : 6.91(s, 1H), 5.68(d, 1H), 5.42(s, 1H), 5.18(d, 1H), 4.42(ABq, 2H), 3.61(s, 3H), 3.48(ABq, 2H), 2.89(d, 3H), 1.50(s, 6H)
IR(KBr, $cm^{-1}$) : 1775, 1633
MS(FAB, M + 1) : 638

Ex. 36: Synthesis of 7-[(Z)-2-(2-aminothiazol-4-yl)-2-(ethoxyimino)acetamido]-3-(4-amino-6-methylamino-1-methylpyrimidinium-2-yl)thiomethyl-3-cephem-4-carboxylate

NMR($\delta$, $D_2O$) : 6.92(s, 1H), 5.82(d, 1H), 5.54(s, 1H), 5.14(d, 1H), 4.40(ABq, 2H), 4.25(q, 2H), 3.54(s, 3H), 3.48(ABq, 2H), 2.91(d, 3H), 1.28(t, 3H)
IR(KBr, $cm^{-1}$) : 1776, 1630
MS(FAB, M + 1) : 580

Ex. 37: synthesis of 7-[(Z)-2-(2-aminothiazol-4-yl)-2-(carboxyprop-2-oxyimino)acetamido]-3-(4-amino-6-ethylamino-1-methylpyrimidinium-2-yl)thiomethyl-3-cephem-4-carboxylate

NMR($\delta$, $D_2O$) : 6.98(s, 1H), 5.80(d, 1H), 5.60(s, 1H), 5.12(d, 1H), 4.42(ABq, 2H), 3.60(s, 3H), 3.40(ABq, 2H), 2.95(q, 2H), 1.51(s, 6H), 1.18(t, 3H)
IR(KBr, $cm^{-1}$) : 1776, 1634
MS(FAB, M + 1) : 652

Ex. 38: Synthesis of 7-[(Z)-2-(2-aminothiazol-4-yl)-2-(ethoxyimino)acetamido]-3-(4-amino-6-ethylamino-1-methylpyrimidinium-2-yl)thiomethyl-3-cephem-4-carboxylate

NMR($\delta$, $D_2O$) : 7.00(s, 1H), 5.88(d, 1H), 5.61(s, 1H), 5.21(d, 1H), 4.45(ABq, 2H), 4.28(q, 2H), 3.61(s, 3H), 3.30(ABq, 2H), 3.00(q, 2H), 1.52(t, 3H), 1.18(t, 3H)

IR(KBr, $cm^{-1}$) : 1774, 1632

MS(FAB, M + 1) : 594

Ex. 39: Synthesis of 7-[(Z)-2-(2-aminothiazol-4-yl)-2-(carboxyprop-2-oxyimino)acetamido]-3-(4-amino-5-carboxy-1-methylpyrimidinium-2-yl)thiomethyl-3-cephem-4-carboxylate

NMR($\delta$, $D_2O$) : 8.20(s, 1H), 6.98(s, 1H), 5.78(d, 1H), 5.12(d, 1H), 4.42(ABq, 2H), 3.62(s, 3H), 3.34(ABq, 2H), 1.54(s, 6H)

IR(KBr, $cm^{-1}$) : 1772, 1632

MS(FAB, M + 1) : 653

Ex. 40: Synthesis of 7-[(Z)-2-(2-aminothiazol-4-yl)-2-(carboxyprop-2-oxyimino)acetamido]-3-(4-amino-5-formamido-1-methylpyrimidinium-2-yl)thiomethyl-3-cephem-4-carboxylate

NMR($\delta$, $D_2O$) : 8.39(s, 1H), 6.95(s, 1H), 5.79(d, 1H), 5.13(d, 1H), 4.42(ABq, 2H), 3.78(s, 3H), 3.64(ABq, 2H), 1.50(s, 6H)

IR(KBr, $cm^{-1}$) : 1776, 1637

MS(FAB, M + 1) : 652

Ex. 41: Synthesis of 7-[(Z)-2-(2-aminothiazol-4-yl)-2-(ethoxyimino)acetamido]-3-(4-amino-5-formamido-1-methylpyrimidinium-2-yl)thiomethyl-3-cephem-4-carboxylate

NMR($\delta$, $D_2O$ + acetone-$d_6$): 8.40(s, 1H), 6.98(s, 1H), 5.82(d, 1H), 5.21(d, 1H), 4.40(ABq, 2H), 4.08(q, 2H), 3.70(s, 3H), 3.48(ABq, 2H), 1.21(t, 3H)

IR(KBr, $cm^{-1}$) : 1774, 1630

MS(FAB, M + 1) : 594

Ex. 42: Synthesis of 7-[(Z)-2-(2-aminothiazol-4-yl)-2-(ethoxyimino)acetamido]-3-(1,4-diaminopyrimidinium-2-yl)thiomethyl-3-cephem-4-carboxylate

NMR($\delta$, $D_2O$ + acetone-$d_6$): 7.95(d, 1H), 7.01(s, 1H), 6.50(d, 1H), 5.80(d, 1H), 5.18(d, 1H), 4.40(ABq, 2H), 4.28(q, 2H), 3.46(ABq, 2H), 1.30(t, 3H)

IR(KBr, $cm^{-1}$) : 1775, 1632

MS(FAB, M + 1) : 552

Ex. 43: Synthesis of 7-[(Z)-2-(2-aminothiazol-4-yl)-2-(carboxyprop-2-oxyimino)acetamido]-3-(1,4-diaminopyrimidinium-2-yl)thiomethyl-3-cephem-4-carboxylate

NMR($\delta$, $D_2O$) : 7.91(d, 1H), 6.99(s, 1H), 6.21(d, 1H), 5.80(d, 1H), 5.24(d, 1H), 4.52(ABq, 2H), 3.40(ABq, 2H), 1.46(s, 6H)

IR(KBr, $cm^{-1}$) : 1773, 1630

MS(FAB, M + 1) : 610

Ex. 44: Synthesis of 7-[(Z)-2-(2-aminothiazol-4-yl)-2-(methoxyimino)acetamido]-3-(1,4-diaminopyrimidinium-2-yl)thiomethyl-3-cephem-4-carboxylate

NMR($\delta$, $D_2O$ + acetone-$d_6$): 7.88(d, 1H), 7.00(s, 1H), 6.29(d, 1H), 5.79(d, 1H), 5.12(d, 1H), 4.40(ABq, 2H), 4.01(s, 3H), 3.34(ABq, 2H)

IR(KBr, $cm^{-1}$) : 1776, 1620

MS(FAB, M + 1) : 538

Ex 45: Synthesis of 7-[(Z)-2-(5-amino-1,2,4-thiadiazol-3-yl)-2-(ethoxyimino)acetamido]-3-(1,4-diaminopyrimidinium-2-yl)thiomethyl-3-cephem-4-carboxylate

NMR($\delta$, $D_2O$ + acetone-$d_6$): 7.90(d, 1H), 6.24(d, 1H), 5.81(d, 1H), 5.28(d, 1H), 4.48(ABq, 2H), 4.21(q, 2H), 3.38(ABq, 2H), 1.48(t, 3H)
IR(KBr, $cm^{-1}$) : 1775, 1631
MS(FAB, M + 1) : 553

Ex. 46: Synthesis of 7-[(Z)-2-(2-aminothiazol-4-yl)-2-(ethoxyimino)acetamido]-3-(1,4,5-triaminopyrimidinium-2-yl)thiomethyl-3-cephem-4-carboxylate

NMR($\delta$, $D_2O$ + acetone-$d_6$): 7.88(s, 1H), 6.98(s, 1H), 5.80(d, 1H), 5.18(d, 1H), 4.42(ABq, 2H), 4.12(q, 2H), 3.34(ABq, 2H), 1.28(t, 3H)
IR(KBr, $cm^{-1}$) : 1776, 1632
MS(FAB, M + 1) : 567

Ex. 47: Synthesis of 7-[(Z)-2-(2-aminothiazol-4-yl)-2-(carboxyprop-2-oxyimino)acetamido]-3-(1,4,5-triaminopyrimidinium-2-yl)thiomethyl-3-cephem-4-carboxylate

NMR($\delta$, $D_2O$) : 7.89(s, 1H), 7.02(s, 1H), 5.82(d, 1H), 5.19(d, 1H), 4.40(ABq, 2H), 3.38(ABq, 2H), 1.50(s, 6H)
IR(KBr, $cm^{-1}$) : 1774, 1630
MS(FAB, M + 1) : 625

Ex. 48: Synthesis of 7-[(Z)-2-(2-aminothiazol-4-yl)-2-(methoxyimino)acetamido]-3-(1,4,5-triaminopyrimidinium-2-yl)thiomethyl-3-cephem-4-carboxylate

NMR($\delta$, $D_2O$ + actone-$d_6$): 7.90(s, 1H), 7.00(s, 1H), 5.80(d, 1H), 5.19(d, 1H), 4.42(ABq, 2H), 4.02(s, 3H), 3.32(ABq, 2H)
IR(KBr, $cm^{-1}$) : 1775, 1631
MS(FAB, M + 1) : 553

Ex. 49: Synthesis of 7-[(Z)-2-(5-amino-1,2,4-thiadiazol-3-yl)-2-(ethoxyimino)acetamido]-3-(1,4,5-triaminopyrimidinium-2-yl)thiomethyl-3-cephem-4-carboxylate

NMR($\delta$, $D_2O$ + acetone-$d_6$): 7.90(s, 1H), 5.82(d, 1H), 5.18(d, 1H), 4.40(ABq, 2H), 4.28(q, 2H), 3.41(ABq, 2H), 1.38(t, 3H)
IR(KBr, $cm^{-1}$) : 1776, 1630
MS(FAB, M + 1) : 568

Ex. 50: Synthesis of 7-[(Z)-2-(2-aminothiazol-4-yl)-2-(carboxyprop-2-oxyimino)acetamido]-3-{4-amino-1-methyl-6-(N,N-dimethyl)aminopyrimidinium-2-yl}thiomethyl-3-cephem-4-carboxylate

NMR($\delta$, $D_2O$) : 7.02(s, 1H), 5.83(d, 1H), 5.62(s, 1H), 5.18(d, 1H), 4.28(ABq, 2H), 3.30(ABq, 2H), 3.52(s, 3H), 3.04(s, 6H), 1.48(s, 6H)
IR(KBr, $cm^{-1}$) : 1774, 1630
MS(FAB, M + 1) : 652

Ex. 51: Synthesis of 7-[(Z)-2-(2-aminothiazol-4-yl)-2-(methoxyimino)acetamido]-3-{4-amino-1-methyl-6-(N,N-dimethyl)aminopyrimidinium-2-yl}thiomethyl-3-cephem-4-carboxylate

NMR($\delta$, $D_2O$ + acetone-$d_6$):
NMR($\delta$, $D_2O$ + acetone-$d_6$): 6.98(s, 1H), 5.80(d, 1H), 5.60(s, 1H), 5.20(d, 1H), 4.40(ABq, 2H), 3.40(ABq, 2H), 3.89(s, 3H), 3.62(s, 3H), 3.02(s, 6H)
IR(KBr, $cm^{-1}$) : 1775, 1632
MS(FAB, M + 1) : 580

Ex. 52: Synthesis of 7-[(Z)-2-(2-aminothiazol-4-yl)-2-(ethoxyimino)acetamido]-3-{4-amino-1-methyl-6-(N,N-dimethyl)aminopyrimidinium-2-yl}thiomethyl-3-cephem-4-carboxylate

NMR(δ, $D_2O$ + acetone-$d_6$): 6.99(s, 1H), 5.81(d, 1H), 5.60(s, 1H), 5.18(d, 1H), 4.38(ABq, 2H), 4.18(q, 2H), 3.38(ABq, 2H), 3.64(s, 3H), 3.00(s, 6H), 1.28(t, 3H)
IR(KBr, $cm^{-1}$) : 1775, 1630
MS(FAB, M + 1) : 594

Ex. 53: Synthesis of 7-[(Z)-2-(2-aminothiazol-4-yl)-2-(carboxyprop-2-oxyimino)acetamido]-3-(1,4,5,6-tetraaminopyrimidinium-2-yl)thiomethyl-3-cephem-4-carboxylate

NMR(δ, $D_2O$) : 7.00(s, 1H), 5.81(d, 1H), 5.20(d, 1H), 4.42(ABq, 2H), 3.30(ABq, 2H), 1.52(s, 6H)
IR(KBr, $cm^{-1}$) : 1775, 1632
MS(FAB, M + 1) : 640

Ex. 54: Synthesis of 7-[(Z)-2-(2-aminothiazol-4-yl)-2-(methoxyimino)acetamido]-3-(1,4,5,6-tetraaminopyrimidinium-2-yl)thiomethyl-3-cephem-4-carboxylate

NMR(δ, $D_2O$) : 7.01(s, 1H), 5.78(d, 1H), 5.18(d, 1H), 4.40(ABq, 2H), 3.98(s, 3H), 3.32(ABq, 2H)
IR(KBr, $cm^{-1}$) : 1774, 1632
MS(FAB, M + 1) : 568

Ex. 55: Synthesis of 7-[(Z)-2-(2-aminothiazol-4-yl)-2-(ethoxyimino)acetamido]-3-(1,4,5,6-tetraaminopyrimidinium-2-yl)thiomethyl-3-cephem-4-carboxylate

NMR(δ, $D_2O$) : 7.02(s, 1H), 5.82(d, 1H), 5.20(d, 1H), 4.43(ABq, 2H), 4.23(q, 2H), 3.34(ABq, 2H), 1.23(t, 3H)
IR(KBr, $cm^{-1}$) : 1775, 1634
MS(FAB, M + 1) : 582

Ex. 56: Synthesis of 7-[(Z)-2-(5-amino-1,2,4-thiadiazol-3-yl)-2-(ethoxyimino)acetamido]-3-(1,4,5,6-tetraaminopyrimidinium-2-yl)thiomethyl-3-cephem-4-carboxylate

NMR(δ, $D_2O$ + acetone-$d_6$): 5.83(d, 1H), 5.21(d, 1H), 4.45(ABq, 2H), 4.38(q, 2H), 3.35(ABq, 2H), 1.43(t, 3H)
IR(KBr, $cm^{-1}$) : 1770, 1632
MS(FAB, M + 1) : 583

Ex. 57: Synthesis of 7-[(Z)-2-(2-aminothiazol-4-yl)-2-(carboxyprop-2-oxyimino)acetamido]-3-(1,4-diamino-5-methylpyrimidinium-2-yl)thiomethyl-3-cephem-4-carboxylate

NMR(δ, $D_2O$) : 7.99(s, 1H), 7.02(s, 1H), 5.80(d, 1H), 5.20(d, 1H), 4.38(ABq, 2H), 3.32(ABq, 2H), 1.92(s, 3H), 1.50(s, 6H)
IR(KBr, $cm^{-1}$) : 1776, 1630
MS(FAB, M + 1) : 624

Ex. 58: Synthesis of 7-[(Z)-2-(2-aminothiazol-4-yl)-2-(carboxymethoxyimino)acetamido]-3-(1,4-diamino-5-methylpyrimidinium-2-yl)thiomethyl-3-cephem-4-carboxylate

NMR(δ, $D_2O$) : 8.02(s, 1H), 7.00(s, 1H), 5.79(d, 1H), 5.20(d, 1H), 4.87(s, 2H), 4.41(ABq, 2H), 3.32(ABq, 2H), 1.92(s, 3H)
IR(KBr, $cm^{-1}$) : 1772, 1635
MS(FAB, M + 1) : 596

Ex. 59: Synthesis of 7-[(Z)-2-(2-aminothiazol-4-yl)-2-(methoxyimino)acetamido]-3-(1,4-diamino-5-methyl-pyrimidinium-2-yl)thiomethyl-3-cephem-4-carboxylate
NMR(δ, $D_2O$ + acetone-$d_6$) : 8.03(s, 1H), 7.01(s, 1H), 5.81(d, 1H), 5.19(d, 1H), 4.42(ABq, 2H), 3.42(ABq, 2H), 3.81(s, 3H), 1.92(s, 3H)

IR(KBr, $cm^{-1}$) : 1774, 1632
MS(FAB, M + 1) : 552

Ex. 60: Synthesis of 7-[(Z)-2-(2-aminothiazol-4-yl)-2-(ethoxyimino)acetamido]-3-(1,4-diamino-5-methyl-pyrimidinium-2-yl)thiomethyl-3-cephem-4-carboxylate

NMR(δ, $D_2O$ + acetone-$d_6$): 8.02(s, 1H), 7.00(s, 1H), 5.82(d, 1H), 5.21(d, 1H), 4.40(ABq, 2H), 3.42(ABq, 2H), 4.12(q, 2H), 1.92(s, 3H), 1.38(t, 3H)
IR(KBr, $cm^{-1}$) : 1775, 1630
MS(FAB, M + 1) : 566

Ex. 61: Synthesis of 7-[(Z)-2-(5-amino-1,2,4-thiadiazol-3-yl)-2-(ethoxyimino)acetamido]-3-(1,4-diamino-5-methyl-pyrimidinium-2-yl)thiomethyl-3-cephem-4-carboxylate

NMR(δ, $D_2O$ + acetone-$d_6$): 8.00(s, 1H), 5.83(d, 1H), 5.21(d, 1H), 4.42(ABq, 2H), 4.28(q, 2H), 3.42(ABq, 2H), 1.92(s, 3H), 1.42(t, 3H)
IR(KBr, $cm^{-1}$) : 1776, 1635
MS(FAB, M + 1) : 567

Ex. 62: Synthesis of 7-[(Z)-2-(2-aminothiazol-4-yl)-2-(carboxyprop-2-oxyimino)acetamido]-3-(1,4-diamino-5-ethylpyrimidinium-2-yl)thiomethyl-3-cephem-4-carboxylate

NMR(δ, $D_2O$) : 7.99(s, 1H), 7.02(s, 1H), 5.80(d, 1H), 5.20(d, 1H), 4.38(ABq, 2H), 3.32(ABq, 2H), 1.90(q, 2H), 1.50(s, 6H), 1.12(t, 3H)
IR(KBr, $cm^{-1}$) : 1776, 1630
MS(FAB, M + 1) : 638

Ex. 63: Synthesis of 7-[(Z)-2-(2-aminothiazol-4-yl)-2-(ethoxyimino)acetamido]-3-(1,4-diamino-5-ethylpyrimidinium-2-yl)thiomethyl-3-cephem-4-carboxylate

NMR(δ, $D_2O$ + acetone-$d_6$): 8.02(s, 1H), 7.00(s, 1H), 5.82(d, 1H), 5.21(d, 1H), 4.40(ABq, 2H), 3.42(ABq, 2H), 4.12(q, 2H), 1.90(q, 2H), 1.38(t, 3H), 1.12(t, 3H)
IR(KBr, $cm^{-1}$) : 1775, 1630
MS(FAB, M + 1) : 580

Ex. 64: Synthesis of 7-[(Z)-2-(2-aminothiazol-4-yl)-2-(methoxyimino)acetamido]-3-(1,4-diamino-5-ethylpyrimidinium-2-yl)thiomethyl-3-cephem-4-carboxylate

NMR(δ, $D_2O$ + acetone-$d_6$): 8.03(s, 1H), 7.01(s, 1H), 5.81(d, 1H), 5.19(d, 1H), 4.42(ABq, 2H), 3.42(ABq, 2H), 3.81(s, 3H), 1.90(q, 2H), 1.10(t, 3H)
IR(KBr, $cm^{-1}$) : 1774, 1632
MS(AB, M+1) : 566

Ex. 65: Synthesis of 7-[(Z)-2-(2-aminothiazol-4-yl)-2-(carboxyprop-2-oxyimino)acetamido]-3-{1,4-diamino-6-(N-methyl)aminopyrimidinium-2-yl}thiomethyl-3-cephem-4-carboxylate

NMR(δ, $D_2O$) : 6.98(s, 1H), 5.79(d, 1H), 5.51(s, 1H), 5.17(d, 1H), 4.22(ABq, 2H), 3.61(ABq, 2H), 2.84(s, 3H), 1.51(d, 6H)
IR(KBr, $cm^{-1}$) : 1775, 1632
MS(AB, M+1) : 639

Ex. 66: Synthesis of 7-[(Z)-2-(2-aminothiazol-4-yl)-2-(ethoxyimino)acetamido]-3-{1,4-diamino-6-(N-methyl)-aminopyrimidinium-2-yl}thiomethyl-3-cephem-4-carboxylate

NMR(δ, $D_2O$ + acetone-$d_6$): 7.00(s, 1H), 5.80(d, 1H), 5.50(s, 1H), 5.19(d, 1H), 4.23(ABq, 2H), 4.08(q, 2H), 3.58(ABq, 2H), 2.85(s, 3H), 1.26(m, 6H)
IR(KBr, $cm^{-1}$) : 1774, 1633
MS(AB, M+1) : 581

Ex. 67: Synthesis of 7-[(Z)-2-(2-aminothiazol-4-yl)-2-(methoxyimino)acetamido]-3-{1,4-diamino-6-(N-methyl)-aminopyrimidinium-2-yl}thiomethyl-3-cephem-4-carboxylate

NMR(δ, $D_2O$ + acetone-$d_6$): 6.98(s, 1H), 5.79(d, 1H), 5.51(s, 1H), 5.17(d, 1H), 4.38(ABq, 2H), 3.89(s, 3H), 3.60(ABq, 2H), 2.84(s, 3H)
IR(KBr, $cm^{-1}$) : 1776, 1634
MS(AB, M+1) : 567

Ex. 68: Synthesis of 7-[(Z)-2-(5-amino-1,2,4-thiadiazol-3-yl)-2-(ethoxyimino)acetamido]-3-{1,4-diamino-6-(N-methyl)aminopyrimidinium-2-yl}thiomethyl-3-cephem-4-carboxylate

NMR(δ, $D_2O$ + acetone-$d_6$): 5.82(d, 1H), 5.52(s, 1H), 5.20(d, 1H), 4.40(ABq, 2H), 4.21(q, 2H), 3.52(ABq, 2H), 2.85(s, 3H), 1.38(t, 3H)
IR(KBr, $cm^{-1}$) : 1776, 1635
MS(AB, M+1) : 582

Ex. 69: Syntheis of 7-[(Z)-2-(2-aminothiazol-4-yl)-2-(carboxymethoxyimino)acetamido]-3-{1,4-diamino-6-(N-methyl)aminopyrimidinium-2-yl}thiomethyl-3-cephem-4-carboxylate
NMR(δ, $D_2O$) : 6.98(s, 1H), 5.81(d, 1H), 5.52(s, 1H), 5.18(d, 1H), 4.84(s, 2H), 4.48(ABq, 2H), 3.60(ABq, 2H), 2.84(s, 3H)
IR(KBr, $cm^{-1}$) : 1776, 1634
MS(AB, M+1) : 611

Ex. 70: Synthesis of 7-[(Z)-2-(2-aminothiazol-4-yl)-2-(carboxyprop-2-oxyimino)acetamido]-3-{1,4-diamino-5-methyl-6-(N-methyl)aminopyrimidinium-2-yl}thiomethyl-3-cephem-4-carboxylate

NMR(δ, $D_2O$) : 7.01(s, 1H), 5.82(d, 1H), 5.20(d, 1H), 4.58(ABq, 2H), 3.45(ABq, 2H), 2.75(s, 3H), 1.92(s, 3H), 1.50(s, 6H)
IR(KBr, $cm^{-1}$) : 1775, 1630
MS(AB, M+1) : 653

Ex. 71: Synthesis of 7-[(Z)-2-(2-aminothiazol-4-yl)-2-(ethoxyimino)acetamido]-3-{1,4-diamino-5-methyl-6-(N-methyl)aminopyrimidinium-2-yl}thiomethyl-3-cephem-4-carboxylate

NMR(δ, $D_2O$ + acetone-$d_6$): 7.00(s, 1H), 5.84(d, 1H), 5.22(d, 1H), 4.50(ABq, 2H), 4.04(q, 2H), 3.40(ABq, 2H), 2.77(s, 3H), 1.92(s, 3H), 1.21(t, 3H)
IR(KBr, $cm^{-1}$) : 1775, 1631
MS(AB, M+1) : 595

Ex. 72: Synthesis of 7-[(Z)-2-(2-aminothiazol-4-yl)-2-(methoxyimino)acetamido]-3-{1,4-diamino-5-methyl-6-(N-methyl)aminopyrimidinium-2-yl}thiomethyl-3-cephem-4-carboxylate

NMR(δ, $D_2O$ + acetone-$d_6$): 6.99(s, 1H), 5.82(d, 1H), 5.19(d, 1H), 4.48(ABq, 2H), 3.92(s, 3H), 3.42(ABq, 2H), 2.74(s, 3H), 1.93(s, 3H)
IR(KBr, $cm^{-1}$) : 1775, 1630
MS(AB, M+1) : 581

Ex. 73: Synthesis of 7-[(Z)-2-(5-amino-1,2,4-thiadiazol-3-yl)-2-(ethoxyimino)acetamido]-3-{1,4-diamino-5-methyl-6-(N-methyl)aminopyrimidinium-2-yl}thiomethyl-3-cephem-4-carboxylate

NMR(δ, $D_2O$ + acetone-$d_6$): 5.80(d, 1H), 5,21(d, 1H), 4.50(ABq, 2H), 4.21(q, 2H), 3.48(ABq, 2H), 2.72(s, 3H), 1.90(s, 3H), 1.38(t, 3H)
IR(KBr, $cm^{-1}$) : 1776, 1632
MS(AB, M+1) : 596

Ex. 74: Synthesis of 7-[(Z)-2-(2-aminothiazol-4-yl)-2-(carboxymethoxyimino)acetamido]-3-{1,4-diamino-5-methyl-6-(N-methyl)aminopyrimidinium-2-yl}thiomethyl-3-cephem-4-carboxylate

NMR(δ, $D_2O$) : 7.00(s, 1H), 5,80(d, 1H), 5.19(d, 1H), 4.82(d, 2H), 4.50(ABq, 2H), 3.45(ABq, 2H), 2.74(s, 3H), 1.92(s, 3H)

IR(KBr, $cm^{-1}$) : 1774, 1632

MS(AB, M+1) : 625

Ex. 75: Synthesis of 7-[(Z)-2-(2-aminothiazol-4-yl)-2-(carboxymethoxyimino)acetamido]-3-(1,4-diaminopyrimidinium-2-yl)thiomethyl-3-cephem-4-carboxylate

NMR(δ, $D_2O$) : 7.78(d, 1H), 7.01(s, 1H), 5.84(d, 1H), 5.82(d, 1H), 5.20 (d, 1H), 4.50(ABq, 2H), 3.48(ABq, 2H)

IR(KBr, $cm^{-1}$) : 1775, 1632

MS(AB, M+1) : 582

Ex. 76: Synthesis of 7-[(Z)-2-(2-aminothiazol-4-yl)-2-(carboxycyclobutoxyimino)acetamido]-3-(1,4-diaminopyrimidinium-2-yl)thiomethyl-3-cephem-4-carboxylate

NMR(δ, $D_2O$ + $NaHCO_3$) : 1.76~2.58(m, 6H), 3.63(ABq, 2H), 4.43(ABq, 2H), 5.18(d, 1H), 5.83(d, 1H), 6.51(d, 1H), 7.01(s, 1H), 7.93(d, 1H)

IR(KBr, $cm^{-1}$) : 1770, 1635

MS(FAB, M+1) : 622

Ex. 77: Synthesis of 7-[(Z)-2-(2-aminothiazol-4-yl)-2-(carboxycyclobutoxyimino)acetamido]-3-(4-amino-1-methylpyrimidinium-2-yl)thiomethyl-3-cephem-4-carboxylate

NMR(δ, $D_2O$ + $NaHCO_3$) : 1.75~2.53(m, 6H), 3.62(ABq, 2H), 3.72(s, 3H), 4.49(ABq, 2H), 5.21(d, 1H), 5.82(d, 1H), 6.51(d, 1H), 7.02(s, 1H), 7.87(d, 1H)

IR(KBr, $cm^{-1}$) : 1765, 1630

MS(FAB, M+1) : 621

Ex. 78: Synthesis of 7-[(Z)-2-(2-aminothiazol-4-yl)-2-(carboxycyclopentoxyimino)acetamido]-3-(1,4-diaminopyrimidinium-2-yl)thiomethyl-3-cephem-4-carboxylate

NMR(δ, $D_2O$ + $NaHCO_3$) : 1.75(m, 4H), 2.01 (m, 4H), 3.60(ABq, 2H), 4.35(ABq, 2H), 5.18(d, 1H), 5.79(d, 1H), 6.51(d, 1H), 7.02(s, 1H), 7.97(d, 1H)

IR(KBr, $cm^{-1}$) : 1770, 1635

MS(FAB, M+1) : 636

Ex. 79: Synthesis of 7-[(Z)-2-(2-aminothiazol-4-yl)-2-(carboxycyclopentoxyimino)acetamido]-3-(4-amino-1-methylpyrimidinium-2-yl)thiomethyl-3-cephem-4-carboxylate

NMR(δ, $D_2O$ + $NaHCO_3$) : 1.77(m, 4H), 2.12(m, 4H), 3.60(ABq, 2H), 3.71(s, 3H), 4.45 (ABq, 2H), 5.19(d, 1H), 5.77(d, 1H), 6.53(d, 1H), 6.99(s, 1H), 7.92(d, 1H)

IR(KBr, $cm^{-1}$) : 1775, 1650

MS(FAB, M+1) : 635

Ex. 80: Synthesis of 7-[(Z)-2-(2-aminothiazol-4-yl)-2-(carboxycyclohexoxyimino)acetamido]-3-(1,4-diaminopyrimidinium-2-yl)thiomethyl-3-cephem-4-carboxylate

NMR(δ, $D_2O$ + $NaHCO_3$) : 1.25~2.12(m, 10H), 3.62(ABq, 2H), 4.37(ABq, 2H), 5.17 (d, 1H), 5.84(d, 1H), 6.52(d, 1H), 7.01(s, 1H), 7.98(d, 1H)

IB(KBr, $cm^{-1}$) : 1770, 1630

MS(FAB, M+1) : 650

Ex. 81: Synthesis of 7-[(Z)-2-(2-aminothiazol-4-yl)-2-(carboxycyclohexoxyimino)acetamido-3-(4-amino-1-methylpyrimidinium-2-yl)thiomethyl-3-cephem-4-carboxylate

NMR(δ, $D_2O$ + $NaHCO_3$) : 1.25~2.15(m, 10H), 3.67(ABq, 2H), 3.73(s, 3H), 4.47(ABq, 2H), 5.18(d, 1H), 5.83(d, 1H), 6.53(d, 1H), 6.97(s, 1H), 7.91(d, 1H)
IR(KBr, $cm^{-1}$) : 1772, 1635
MS(FAB, M+1) : 649

Ex. 82: Synthesis of 7-[(Z)-2-(2-aminothiazol-4-yl)-2-{1-carboxyeth-1-oxyimino}acetamido]-3-(4,5,6-triamino-1-methylpyrimidinium-2-yl)thiomethyl-3-cephem-4-carboxylate(82-a)and (82-b)

To a solution of 1.00g of paramethoxybenzyl 3-chloromethyl-7-[(Z)-2-{2-(triphenylmethyl)aminothiazol-4-yl}-2-{(R,S)-1-tert-butoxycarbonyleth-1-oxyimino)acetamido]-3-cephem-4-carboxylate dissolved in 5ml of DMF was added 0.17g of 4,5,6-triamino-1-methyl-2-pyrimidinethione synthesized in Preparation Example 1 above and the reaction solution was stirred at a room temperature for 1 hour. After the reaction was completed, the reaction mixture was distilled under reduced pressure to remove the DMF solvent. The residues so obtained was dissolved in 15ml of $CH_2Cl_2$ and the resultant solution was added slowly to 100ml of ethyl ether to produce white precipitates, which were collected by filtration, dried and dissolved in a mixed solvent of 5ml of anisole and 5ml of trifluoroacetic acid at -30°C. The resulting solution was stirred at room temperature for 1.5 hours and thereto was added 40 ml of ethyl ether to produce white precipitates, which were washed with 20 ml of acetone and 20 ml of ethyl ether, dried completely and purified by a fractional liquid chromatograply(μ-Bondapak $C_{18}$ steel column, 19mm × 30cm) using as an eluent 5% aqueous methanol solution to obtain 100mg and 120mg of the title compound as a S-isomer(82-a) and R-isomer(82-b), respectively.
(S-isomer)
NMR(δ, $D_2O$ + $NaHCO_3$) : 1.52(d, 3H), 3.68(s, 3H), 3.60(ABq, 2H), 4.32(ABq, 2H), 5.11(d, 1H), 5.78(d, 1H), 6.99(s, 1H)
(R-isomer)
NMR(δ, $D_2O$) : 6.99(s, 1H), 5.76(d, 1H), 5.10(d, 1H), 4.33(ABq, 2H), 3.58(ABq, 2H), 3.68(s, 3H), 1.52(d, 3H)
IR(KBr, $cm^{-1}$) : 1770, 1631
MS(FAB, M + 1) : 625

Example 83 to 87

Simlarly to the procedures described in the above Example 82, the following compounds were synthesized.

Ex. 83: Synthesis of 7-[(Z)-2-(2-aminothiazol-4-yl)-2-{1-carboxyeth-1-oxyimino}acetamido]-3-(4-amino-1-methylpyrimidinium-2-yl)thiomethyl-3-cephem-4-carboxylate

(S-isomer)
NMR(δ, $D_2O$ + $NaHCO_3$) : 1.46(d, 3H), 3.71(s, 3H), 3.58(ABq, 2H), 4.40(ABq, 2H), 4.68(q, 1H), 5.13(d, 1H), 5.78(d, 1H), 6.48(d, 1H), 7.00 (s, 1H), 7.82(d, 1H)
(R-isomer)
NMR(δ, $D_2O$) : 7.82(d, 1H), 7.00(s, 1H), 6.48(d, 1H), 5.77(d, 1H), 5.12(d, 1H), 4.40(ABq, 2H), 3.58(ABq, 2H), 3.71(s, 3H), 1.46(d, 3H)
IR(KBr, $cm^{-1}$) : 1774, 1630
MS(FAB, M + 1) : 595

Ex. 84: Synthesis of 7-[(Z)-2-(2-aminothiazol-4-yl)-2-{1-carboxyeth-1-oxyimino}acetamido]-3-(1,4-diaminopyrimidinium-2-yl)thiomethyl-3-cephem-4-carboxylate

(S-isomer)
NMR(δ, $D_2O$) : 7.90(d, 1H), 7.02(s, 1H), 6.28(d, 1H), 5.78(d, 1H), 5.18(d, 1H), 4.41(ABq, 2H), 3.30(ABq, 2H), 1.50(d, 3H)
(R-isomer)
NMR(δ, $D_2O$) : 7.90(d, 1H), 7.02(s, 1H), 6.28(d, 1H), 5.79(d, 1H), 5.17(d, 1H), 4.40(ABq, 2H), 3.30(ABq,

2H), 1.52(d, 3H)
IR(KBr, $cm^{-1}$) : 1776, 1628
MS(FAB, M + 1) : 596

Ex. 85: Synthesis of 7-[(Z)-2-(2-aminothiazol-4-yl)-2-{1-carboxyeth-1-oxyimino}acetamido]-3-(1,4-diamino-5-methylpyrimidinium-2-yl)thiomethyl-3-cephem-4-carboxylate

NMR(δ, $D_2O$):
(S-isomer)
8.01(s, 1H), 7.00(s, 1H), 5.81(d, 1H), 5.19(d, 1H), 4.40(ABq, 2H), 3.30(ABq, 2H), 1.90(sd, 3H), 1.42(d, 3H)
(R-isomer)
8.02(s, 1H), 7.00(s, 1H), 5.82(d, 1H), 5.20(d, 1H), 4.42(ABq, 2H), 3.34(ABq, 2H), 1.90(s, 3H), 1.42(d, 3H)
IR(KBr, $cm^{-1}$): 1775, 1632
MS(FAB, M+1): 610

Ex. 86: Synthesis of 7-[(Z)-2-(2-aminothiazol-4-yl)-2-{1-carboxyeth-1-oxyimino}acetamido]-3-(1,4-diamino-5-methyl-6-(N-methyl)-aminopyrimidinium-2-yl}thiomethyl-3-cephem-4-carboxylate

NMR(δ, $D_2O$):
(S-isomer)
6.98(s, 1H), 5.81(d, 1H), 5.91(d, 1H), 4.48(ABq, 2H), 3.40(ABq, 2H), 2.78(s, 3H), 1.90(s, 3H), 1.50(d, 3H)
(R-isomer)
6.99(s, 1H), 5.82(d, 1H), 5.19(d, 1H), 4.46(ABq, 2H), 3.42(ABq, 2H), 2.79(s, 3H), 1.90(s, 3H), 1.50(d, 3H)
IR(KBr, $cm^{-1}$): 1775, 1632
MS(FAB, M+1): 639

Ex. 87: Synthesis of 7-[(Z)-2-(2-aminothiazol-4-yl)-2-{1-carboxyeth-1-oxyimino}acetamido]-3-{1,4-diamino-6-(N-methyl)amino-pyrimidinium-2-yl}thiomethyl-3-cephem-4-carboxylate

NMR (δ, $D_2O$):
(S-isomer)
6.99(s, 1H), 5.80(d, 1H), 5.50(d, 1H), 5.19(d, 1H), 4.40(ABq, 2H), 3.48(ABq, 2H), 2.86(s, 3H), 1.48(d, 3H)
(R-isomer)
7.00(s, 1H), 5.81(d, 1H), 5.52(s, 1H), 5.19(d, 1H), 4.43(ABq, 2H), 3.46(ABq, 2H), 2.86(s, 3H), 1.49(d, 3H)
IR(KBr, $cm^{-1}$): 1772, 1635
MS(FAB, M+1): 625

Ex. 88-a: Synthesis of 7-[(Z)-2-(2-aminothiazol-4-yl)-2-{(S)-1-carboxyprop-1-oxyimino}acetamido]-3-(1,4-diaminopyrimidinium-2-yl)thiomethyl-3-cephem-4-carboxylate

To a solution of 2.0g of the compound synthesized Preparation Example 31 above dissolved in 15ml of DMSO was added 350mg of 1,4-diamino-2-pyrimidinethione synthesized in Preparation Example 19 above and the reaction solution was stirred at a room temperature for 3 hours, extracted with a mixed solvent of 30ml of THF and 30ml of ethyl acetate and washed with 150ml of saturated sodium chloride solution. The organic layer was separated therefrom, dried over anhydrous magnesium sulfate, and evaporated under a reduced pressure to remove the solvent. The residues so obtained was dissolved in 6ml of anisole, and the resultant solution was cooled to a temperature ranging from 0°C to 4°C, and 10ml of trifluoroacetic acid was added thereto. The resulting solution was stirred at a room temperature for 40minutes and cooled to a temperature ranging from -20 to -30°C and thereto was added 80ml of diethyl ether to produce white precipitates, which were washed with 70ml of acetone and dried completely to obtain 1.2g of ivory solides. The solids were purified by a fractional liquid chromatography(μ-Bondapak $C_{18}$ steel Colum 19mm × 30cm) using as an eluent 10% methanol aqueous solution to obtain 265mg of the title compound(88-a).
IR(KBr, $cm^{-1}$): 1775(β-lactam), 1675, 1630, 1580
MS(FAB, M+1): 582
NMR(δ, $D_2O$ + $NaHCO_3$) : 0.99(t, 3H), 1.89(q, 2H), 3.56(ABq, 2H), 4.30(ABq, 2H), 4.52 (q, 1H), 5.15(d, 1H), 5.78(d, 1H), 6.49(d, 1H), 7.00(s, 1H), 7.93(d, 1H)
$[\alpha]^{20}_D$: -198.16(c = 0.0103, $H_2O$)

EX. 88-b: Synthesis of 7-[(Z)-2-(2-aminothiazol-4-yl)-2-{(R)-1-carboxyprop-1-oxyimino}acetamido]-3-(1,4-diaminopyrimidinium-2-yl)thiomethyl-3-cephem-4-carboxylate

The same procedures as descibed in Example 88-a above were repeated except that the compound synthesized in Preparative Example 31 was sabstituted with the compound synthesized in Preparative Example 30 to obtain 270 mg of the title compound(88-b) as a white solid.

NMR(δ, $D_2O$ + $NaHCO_3$) : 0.98(t, 3H), 1.84(q, 2H), 3.53(ABq, 2H), 4.26(ABq, 2H), 4.48 (q, 1H), 5.14(d, 1H), 5.74(d, 1H), 6.43(d, 1H), 6.96(s, 1H), 7.91(d, 1H)

$[\alpha]^{20}_D$: -119.35(c = 0.0102, $H_2O$)

Example 89-a: Synthesis of 7-[(Z)-2-(2-aminothiazol-4-yl)-2-{(S)-1-carboxyprop-1-oxyimino}acetamido]-3-(4-amino-1-methylpyrimidinium-2-yl)thiomethyl-3-cephem-4-carboxylate

The same procedures as described in Example 88-a above were repeated except that 1,4-diamino-2-pyrimidinethione was substituted with 4-amino-1-methyl-2-pyrimidinethione synthesized in Preparation Example 2 above to obtain 260mg of the title compound as a white solid.

IR(KBr, $cm^{-1}$): 1770(β-lactam), 1680, 1620, 1570

MS(FAB, M + 1): 581

NMR(δ, $D_2O$ + $NaHCO_3$) : 0.99(t, 3H), 1.87(q, 2H), 3.52(ABq, 2H), 3.55(s, 3H), 4.27 (ABq, 2H), 4.49(q, 1H), 5.15(d, 1H), 5.76(d, 1H), 6.46(d, 1H), 6.96(s, 1H), 7.92(d, 1H)

$[\alpha]^{20}_D$: -189.46(c = 0.0097, $H_2O$)

Example 89-b: Synthesis of 7-[(Z)-2-(2-aminothiazol-4-yl)-2-{(R)-1-carboxyprop-1-oxyimino}acetamido]-3-(4-amino-1-methylpyrimidinium-2-yl)thiomethyl-3-cephem-4-carboxylate

The same procedures as described in Example 88-b above were repeated except that 1,4-diamino-2-pyrimidinethione was substituted with 4-amino-1-methyl-2-pyrimidinethione synthesized in Preparation Example 2 above to obtain 265 mg of the title compound as a white solid(89-b).

NMR(δ, $D_2O$ + $NaHCO_3$) : 0.98(t, 3H), 1.89(q, 2H), 3.53(ABq, 2H), 3.56(s, 3H), 4,29 (ABq, 2H), 4.47(q, 1H), 5.13(d, 1H), 5.75(d, 1H), 6.44(d, 1H), 6.97(s, 1H), 7.92(d, 1H),

$[\alpha]^{20}_D$: -123.24(c = 0.0096, $H_2O$)

Example 90: Synthesis of 7-[(Z)-2-(2-aminothiazol-4-yl)-2-(2-carboxybut-2-oxyimino)acetamido]-3-(1,4-diaminopyrimidinium-2-yl)thiomethyl-3-cephem-4-carboxylate

The sample procedures as described in Example 88-a above were repeated except that the compound synthesized in Preparation Example 32 was used to obtain 190mg and 195mg of the title compounds as a S-isomer(90-a) and R-isomer(90-b) respectively.

NMR(δ, $D_2O$ + $NaHCO_3$):

(S-isomer)

0.87(t, 3H), 1.48(s, 3H), 1.87(q, 2H), 3.54(ABq, 2H), 4.29(ABq, 2H), 5.12(d, 1H), 5.78(d, 1H), 6.47(d, 1H), 6.96(s, 1H), 7.94(d, 1H)

(R-isomer)

0.87(t, 3H), 1.44(s, 3H), 1.86(q, 2H), 3.55(ABq, 2H), 4.33(ABq, 2H), 5.13(d, 1H), 5.75(d, 1H), 6.47(d, 1H), 6.95(s, 1H), 7.91(d, 1H)

IR(KBr, $cm^{-1}$): 1770(β-lactam ), 1660, 1630, 1570

$[\delta]^{20}_D$:

S-isomer: -178.67(c = 0.0103, $H_2O$)

R-isomer: -135.80(c = 0.0099, $H_2O$)

Example 91: Synthesis of 7-[(Z)-2-(2-aminothiazol-4-yl)-2-(2-carboxybut-2-oxyimino)acetamido]-3-(4-amino-1-methylpyrimidinium-2-yl)thiomethyl-3-cephem-4-carboxylate

The same procedures as described in Example 90 above were repeated except that 1,4-diamino-2-pyrimidinethione was substituted with 4-amino-1-methyl-2-pyrimidinethione synthesized in Preparation Example 2 to obtain 185mg and 187mg of the title compounds as a S-isomer(91-a) and R-iosomer(91-b), respectively.

NMR(δ, $D_2O$ + $NaHCO_3$):

(S-isomer)

0.88(t, 3H), 1.46(s, 3H), 1.87(q, 2H), 3.55(ABq, 2H), 3.57(s, 3H), 4.30(ABq, 2H), 5.12(d, 1H), 5.74(d, 1H), 6.46(d, 1H), 6.96(s, 1H), 7.92(d, 1H)

(R-isomer)

0.87(t, 3H), 1.47(s, 3H), 1.86(q, 2H), 3.54(ABq, 2H), 3.56(s, 3H), 4.29(ABq, 2H), 5.13(d, 1H), 5.76(d, 1H), 6.45(d, 1H), 6.97(s, 1H), 7.91(d, 1H)

IR(KBr, $cm^{-1}$): 1775($\beta$-lactam ), 1650, 1640, 1560

$[\delta]^{20}{}_{D}$:

S-isomer: -167.49(c = 0.0102, $H_2O$)

R-isomer: -128.83(c = 0.0105 , $H_2O$)

Activity Test

In order to illustrate surprisingly superior antibacterial effectiveness of the compounds of the present invention, the minimal inhibitory concentrations(MIC) of the compounds synthesized against standard strains were determined and compared with Ceftazidime, which were used as the control compounds.

These MIC values were taken by employing a two-fold dilution method: that is, two-fold serial dilutions of each of the test compounds were made and dispersed in a Muller-Hinton agar medium; 2$\mu$l of the standard test strain which had the $10^7$ CFU(Colony Forming Unit) per ml was inoculated on the medium; and these were incubated at 37°C for 20 hours. The results of the MIC tests are shown in Table 2.

Table 2. MIC values against the standard strains(μg/ml)

| Organisms | Example No. | 1 | 2 | 3 | 4 | 5 | 6 | 7 | 8 |
|---|---|---|---|---|---|---|---|---|---|
| *Bacillus cereus* | ATCC 11778 | 128 | 64 | 32 | 32 | 16 | 16 | 16 | 64 |
| *Bacillus megaterium* | ATCC 9885 | 0.25 | 0.25 | 0.031 | 0.13 | 0.063 | 0.063 | 0.13 | 0.13 |
| *Micrococcus luteus* | ATCC 9341 | 0.25 | 0.25 | 0.031 | 0.063 | 0.031 | 0.063 | 0.063 | 0.13 |
| *Staphylococcus aureus* | ATCC 6538p | 8 | 4 | 0.5 | 0.5 | 0.5 | 0.13 | 0.13 | 0.5 |
| *Staphylococcus aureus* | ATCC 10537 | 2 | 2 | 0.25 | 0.25 | 0.25 | 0.13 | 0.13 | 0.25 |
| *Staphylococcus epidermidis* | ATCC 12228 | 2 | 2 | 0.13 | 0.13 | 0.25 | 0.13 | 0.13 | 0.13 |
| *Streptococcus faecalis* | ATCC 29212 | >128 | >128 | 64 | 64 | 32 | 32 | 128 | 64 |
| *Acinetobacter calcoaceticus* | ATCC 15437 | 8 | 4 | 8 | 8 | 2 | 1 | 4 | 8 |
| *Citrobacter freundii* | ATCC 8090 | 0.13 | 0.031 | 0.063 | 0.063 | 0.13 | 0.063 | 0.063 | 0.063 |
| *Enterobacter aerogenes* | ATCC 29751 | 2 | 1 | 0.5 | 0.25 | 0.016 | 0.016 | <=0.008 | <=0.008 |
| *Enterobacter cloacae* | ATCC 27508 | 0.016 | <=0.008 | 0.016 | <=0.008 | 0.13 | 0.063 | 0.063 | <=0.008 |
| *Escherichia coli* | ATCC 10536 | 0.13 | 0.031 | 0.063 | 0.063 | 0.063 | 0.063 | 0.063 | 0.063 |
| *Escherichia coli* | ATCC 25922 | 0.063 | 0.031 | 0.063 | 0.063 | 0.063 | 0.063 | 0.063 | 0.063 |
| *Klebsiella pneumoniae* | ATCC 10031 | 0.063 | 0.031 | <=0.008 | <=0.008 | 0.016 | <=0.008 | 0.016 | 0.016 |
| *Morganella morganii* | ATCC 8076h | <=0.008 | <=0.008 | <=0.008 | <=0.008 | <=0.008 | <=0.008 | <=0.008 | <=0.008 |
| *Proteus mirabilis* | ATCC 25933 | 0.063 | 0.031 | 0.25 | 0.5 | 0.5 | 0.5 | 0.13 | 0.25 |
| *Proteus vulgaris* | ATCC 6059 | 0.063 | 0.063 | 0.25 | 0.5 | 0.5 | 0.5 | 0.13 | 0.25 |
| *Providencia rettgeri* | ATCC 9250 | <=0.008 | <=0.008 | <=0.008 | <=0.008 | <=0.008 | <=0.008 | <=0.008 | <=0.008 |
| *Salmonella typhimurium* | ATCC 14028 | 0.25 | 0.13 | 0.5 | 0.25 | 0.25 | 0.5 | 0.13 | 0.25 |
| *Serratia marcescens* | ATCC 27117 | 0.25 | 0.063 | 0.25 | 0.25 | 0.25 | 0.5 | 0.13 | 0.25 |
| *Shigella flexneri* | ATCC 11836 | 0.031 | 0.016 | 0.063 | 0.031 | 0.031 | 0.063 | 0.031 | 0.063 |
| *Shigella sonnei* | ATCC 11060 | 0.13 | 0.063 | 0.25 | 0.25 | 0.25 | 0.25 | 0.13 | 0.13 |
| *Pseudomonas aeruginosa* | ATCC 25619 | 1 | 1 | 0.5 | 1 | 0.5 | 0.5 | 4 | 8 |
| *Pseudomonas aeruginosa* | ATCC 27853 | 1 | 1 | 4 | 8 | 2 | 2 | 32 | 64 |
| *Pseudomonas aeruginosa* | ATCC 10145 | 2 | 2 | 8 | 16 | 4 | 4 | 32 | 64 |

Table 2(continued)

| Organisms \ Example No. | | 9 | 10 | 11 | 12 | 13 | 14 | 15 | 16 | 17 | 18 |
|---|---|---|---|---|---|---|---|---|---|---|---|
| *Bacillus cereus* | ATCC 11778 | 16 | 32 | 128 | 128 | 64 | 32 | 32 | 64 | 128 | 32 |
| *Bacillus megaterium* | ATCC 9885 | 0.063 | 0.13 | 0.13 | 0.5 | 0.13 | 0.25 | 0.13 | 0.5 | 0.25 | 0.13 |
| *Micrococcus luteus* | ATCC 9341 | 0.031 | 0.25 | 0.13 | 0.5 | 0.13 | 0.25 | 0.063 | 0.5 | 0.5 | 0.016 |
| *Staphylococcus aureus* | ATCC 6538p | 0.25 | 0.5 | 4 | 4 | 0.5 | 0.5 | 0.5 | 0.25 | 4 | 0.5 |
| *Staphylococcus aureus* | ATCC 10537 | 0.13 | 0.25 | 2 | 2 | 0.25 | 0.25 | 0.25 | 0.5 | 1 | 0.25 |
| *Staphylococcus epidermidis* | ATCC 12228 | 0.063 | 0.063 | 2 | 2 | 0.13 | 0.25 | 0.25 | 0.5 | 1 | 0.13 |
| *Streptococcus faecalis* | ATCC 29212 | 2 | 8 | >128 | 64 | >128 | >128 | 32 | 32 | >128 | 16 |
| *Acinetobacter calcoaceticus* | ATCC 15437 | 0.13 | 0.063 | 8 | 8 | 16 | 16 | 4 | 8 | 2 | 8 |
| *Citrobacter freundii* | ATCC 8090 | 0.016 | 0.063 | 0.13 | 0.13 | 0.5 | 0.13 | 0.5 | 0.5 | 0.13 | 0.13 |
| *Enterobacter aerogenes* | ATCC 29751 | 0.016 | 0.063 | 2 | 2 | 2 | 1 | 0.063 | 0.063 | 4 | 1 |
| *Enterobacter cloacae* | ATCC 27508 | <=0.008 | 0.016 | 0.031 | 0.031 | 0.016 | 0.13 | 0.031 | 0.063 | 0.016 | <=0.008 |
| *Escherichia coli* | ATCC 10536 | 0.031 | 0.25 | 0.031 | 0.063 | 0.063 | 0.13 | 0.063 | 0.031 | 0.063 | 0.13 |
| *Escherichia coli* | ATCC 25922 | 0.031 | 0.25 | 0.063 | 0.063 | 0.063 | 0.063 | 0.063 | 0.031 | 0.13 | 0.25 |
| *Klebsiella pneumoniae* | ATCC 10031 | 0.031 | 0.25 | 0.063 | <=0.008 | 0.13 | 0.13 | 0.016 | 0.016 | 0.063 | <=0.008 |
| *Morganella morganii* | ATCC 8076h | <=0.008 | 0.016 | <=0.008 | 0.016 | 0.13 | 0.13 | <=0.008 | 0.031 | 0.016 | <=0.008 |
| *Proteus mirabilis* | ATCC 25933 | 0.031 | 0.25 | 0.25 | 1 | 2 | 2 | 1 | 2 | 0.063 | 0.5 |
| *Proteus vulgaris* | ATCC 6059 | 0.063 | 1 | 0.25 | 1 | 2 | 2 | 1 | 2 | 0.13 | 0.5 |
| *Providencia rettgeri* | ATCC 9250 | <=0.008 | <=0.008 | 0.063 | <=0.008 | 0.13 | 0.063 | <=0.008 | 0.031 | 0.13 | <=0.008 |
| *Salmonella typhimurium* | ATCC 14028 | 0.25 | 0.5 | 1 | 1 | 1 | 1 | 0.5 | 1 | 0.25 | 0.5 |
| *Serratia marcescens* | ATCC 27117 | 0.25 | 0.5 | 1 | 0.5 | 1 | 1 | 0.5 | 1 | 0.25 | 0.5 |
| *Shigella flexneri* | ATCC 11836 | 0.063 | 0.016 | 0.063 | 0.25 | 0.5 | 0.25 | 0.25 | 0.5 | 0.031 | 0.13 |
| *Shigella sonnei* | ATCC 11060 | 0.25 | 1 | 1 | 0.5 | 0.5 | 0.25 | 0.25 | 0.5 | 0.13 | 0.25 |
| *Pseudomonas aeruginosa* | ATCC 25619 | 64 | 128 | 2 | 2 | 8 | 8 | 1 | 2 | 1 | 2 |
| *Pseudomonas aeruginosa* | ATCC 27853 | 128 | >128 | 4 | 8 | 32 | 32 | 2 | 4 | 2 | 16 |
| *Pseudomonas aeruginosa* | ATCC 10145 | 128 | >128 | 8 | 16 | 64 | 64 | 8 | 16 | 4 | 16 |

Table 2(continued)

| Organisms | Example No. | 19 | 20 | 21 | 25 | 29 | 31 | 32 | 33 | 35 | 36 |
|---|---|---|---|---|---|---|---|---|---|---|---|
| Bacillus cereus | ATCC 11778 | 128 | 64 | 128 | 64 | 16 | 32 | 128 | 32 | 128 | 32 |
| Bacillus megaterium | ATCC 9885 | 0.25 | 0.13 | 0.13 | 0.25 | 0.063 | 0.13 | 0.13 | 0.063 | 0.5 | 0.063 |
| Micrococcus luteus | ATCC 9341 | 0.13 | 0.25 | 0.5 | 0.063 | 0.016 | 0.031 | 0.25 | 0.031 | 0.25 | ≤0.008 |
| Staphylococcus aureus | ATCC 6538p | 4 | 4 | 4 | 1 | 0.13 | 0.13 | 4 | 0.25 | 4 | 0.25 |
| Staphylococcus aureus | ATCC 10537 | 2 | 2 | 1 | 0.5 | 0.13 | 0.13 | 2 | 0.25 | 2 | 0.13 |
| Staphylococcus epidermidis | ATCC 12228 | 2 | 1 | 2 | 0.5 | 0.031 | 0.063 | 2 | 0.13 | 2 | 0.031 |
| Streptococcus faecalis | ATCC 29212 | 64 | >128 | >128 | >128 | 16 | 16 | >128 | 8 | >128 | 32 |
| Acinetobacter calcoaceticus | ATCC 15473 | 16 | 64 | 32 | 32 | 16 | 16 | 8 | 8 | 8 | 4 |
| Citrobacter freundii | ATCC 8090 | 0.063 | 0.5 | 0.13 | 0.063 | 0.13 | 0.25 | 0.13 | 0.063 | 0.063 | 0.016 |
| Enterobacter aerogenes | ATCC 29751 | 1 | 4 | 2 | 1 | 0.25 | 0.5 | 2 | 0.5 | 1 | 0.13 |
| Enterobacter cloacae | ATCC 27508 | ≤0.008 | 0.13 | 0.016 | ≤0.008 | ≤0.008 | 0.016 | 0.016 | ≤0.008 | ≤0.008 | ≤0.008 |
| Escherichia coli | ATCC 10536 | 0.063 | 0.25 | 0.13 | 0.031 | 0.13 | 0.25 | 0.063 | 0.063 | 0.063 | 0.031 |
| Escherichia coli | ATCC 25922 | 0.063 | 0.5 | 0.13 | 0.031 | 0.13 | 0.13 | 0.063 | 0.063 | 0.063 | 0.063 |
| Klebsiella pneumoniae | ATCC 10031 | 0.031 | 0.13 | 0.031 | 0.016 | ≤0.008 | ≤0.008 | 0.016 | ≤0.008 | 0.063 | ≤0.008 |
| Morganella morganii | ATCC 8076h | ≤0.008 | ≤0.008 | ≤0.008 | ≤0.008 | ≤0.008 | 0.016 | ≤0.008 | ≤0.008 | ≤0.008 | 0.016 |
| Proteus mirabilis | ATCC 25933 | 0.063 | 0.25 | 0.25 | 0.25 | 0.5 | 1 | 0.13 | 0.5 | 0.031 | 0.25 |
| Proteus vulgaris | ATCC 6059 | 0.063 | 0.5 | 0.25 | 0.25 | 0.5 | 1 | 0.13 | 0.25 | 0.063 | 0.25 |
| Providencia rettgeri | ATCC 9250 | ≤0.008 | ≤0.008 | ≤0.008 | ≤0.008 | ≤0.008 | 0.016 | ≤0.008 | ≤0.008 | ≤0.008 | ≤0.008 |
| Salmonella typhimurium | ATCC 14028 | 0.25 | 1 | 0.5 | 0.13 | 0.5 | 1 | 0.25 | 0.5 | 0.13 | 0.13 |
| Serratia marcescens | ATCC 27117 | 0.25 | 2 | 0.5 | 0.5 | 0.5 | 1 | 0.25 | 0.5 | 0.13 | 0.13 |
| Shigella flexneri | ATCC 11836 | 0.031 | 0.13 | 0.063 | 0.016 | 0.063 | 0.13 | 0.031 | 0.063 | 0.031 | ≤0.008 |
| Shigella sonnei | ATCC 11060 | 0.063 | 0.25 | 0.13 | 0.063 | 0.25 | 0.25 | 0.13 | 0.13 | 0.063 | 0.063 |
| Pseudomonas aeruginosa | ATCC 25619 | 8 | 2 | 2 | 2 | 4 | 4 | 1 | 0.5 | 2 | 0.5 |
| Pseudomonas aeruginosa | ATCC 27853 | 16 | 4 | 8 | 32 | 16 | 16 | 2 | 8 | 2 | 4 |
| Pseudomonas aeruginosa | ATCC 10145 | 32 | 16 | 16 | 32 | 16 | 32 | 4 | 16 | 2 | 8 |

Table 2(continued)

| Organisms | Example No. | 42 | 43 | 46 | 47 | 50 | 57 | 75 | 82-b | 88-b | Ceftazidime |
|---|---|---|---|---|---|---|---|---|---|---|---|
| Bacillus cereus | ATCC 11778 | 32 | 128 | 64 | 128 | 128 | 128 | 128 | 128 | 128 | 128 |
| Bacillus megaterium | ATCC 9885 | 0.063 | 0.5 | 0.063 | 0.5 | 0.25 | 0.25 | 0.5 | 0.5 | 1 | 0.25 |
| Micrococcus luteus | ATCC 9341 | ≤0.008 | 0.5 | 0.031 | 0.25 | 0.25 | 0.25 | 2 | 0.5 | 1 | 0.5 |
| Staphylococcus aureus | ATCC 6538p | 0.13 | 8 | 0.25 | 4 | 8 | 8 | 8 | 8 | 4 | 8 |
| Staphylococcus aureus | ATCC 10537 | 0.13 | 2 | 0.13 | 2 | 4 | 2 | 4 | 2 | 4 | 4 |
| Staphylococcus epidermidis | ATCC 12228 | 0.031 | 2 | 0.031 | 2 | 2 | 2 | 4 | 2 | 2 | 4 |
| Streptococcus faecalis | ATCC 29212 | 8 | >128 | 8 | >128 | >128 | >128 | >128 | >128 | >128 | >128 |
| Acinetobacter calcoaceticus | ATCC 15473 | 4 | 2 | 4 | 16 | 4 | 16 | 4 | 8 | 8 | 2 |
| Citrobacter freundii | ATCC 8090 | 0.063 | 0.063 | 0.063 | 0.031 | 0.063 | 0.031 | 0.031 | 0.063 | 0.063 | 0.13 |
| Enterobacter aerogenes | ATCC 29751 | 0.13 | 0.5 | 1 | 1 | 0.5 | 0.5 | 0.5 | 0.5 | 1 | 8 |
| Enterobacter cloacae | ATCC 27508 | ≤0.008 | ≤0.008 | ≤0.008 | ≤0.008 | ≤0.008 | ≤0.008 | ≤0.008 | ≤0.008 | 0.016 | 0.031 |
| Escherichia coli | ATCC 10536 | 0.031 | 0.063 | 0.031 | 0.031 | 0.063 | 0.063 | 0.016 | 0.031 | 0.063 | 0.13 |
| Escherichia coli | ATCC 25922 | 0.031 | 0.063 | 0.031 | 0.031 | 0.063 | 0.063 | 0.016 | 0.031 | 0.031 | 0.13 |
| Klebsiella pneumoniae | ATCC 10031 | ≤0.008 | 0.063 | 0.063 | 0.031 | 0.063 | 0.063 | 0.016 | 0.031 | 0.031 | 0.13 |
| Morganella morganii | ATCC 8076h | 0.25 | ≤0.008 | 0.016 | ≤0.008 | ≤0.008 | ≤0.008 | ≤0.008 | ≤0.008 | ≤0.008 | ≤0.008 |
| Proteus mirabilis | ATCC 25933 | 1 | 0.031 | 0.25 | 0.063 | 0.031 | 0.031 | 0.063 | 0.031 | 0.013 | 0.063 |
| Proteus vulgaris | ATCC 6059 | 1 | 0.031 | 0.25 | 0.063 | 0.031 | 0.031 | 0.031 | 0.031 | 0.013 | 0.063 |
| Providencia rettgeri | ATCC 9250 | 0.063 | ≤0.008 | 0.031 | ≤0.008 | ≤0.008 | ≤0,008 | ≤0.008 | ≤0.008 | ≤0.008 | ≤0.008 |
| Salmonella typhimurium | ATCC 14028 | 0.5 | 0.13 | 0.25 | 0.13 | 0.13 | 0.13 | 0.063 | 0.063 | 0.25 | 0.25 |
| Serratia marcescens | ATCC 27117 | 0.5 | 0.13 | 0.25 | 0.13 | 0.13 | 0.13 | 0.031 | 0.063 | 0.25 | 0.13 |
| Shigella flexneri | ATCC 11836 | 0.25 | 0.031 | 0.031 | 0.031 | 0.031 | 0.063 | 0.016 | 0.031 | 0.031 | 0.031 |
| Shigella sonnei | ATCC 11060 | 0.25 | 0.063 | 0.031 | 0.031 | 0.063 | 0.063 | 0.016 | 0.63 | 0.063 | 0.13 |
| Pseudomonas aeruginosa | ATCC 25619 | 0.5 | 1 | 0.5 | 1 | 1 | 1 | 4 | 1 | 1 | 1 |
| Pseudomonas aeruginosa | ATCC 27853 | 4 | 1 | 4 | 2 | 1 | 2 | 4 | 1 | 2 | 1 |
| Pseudomonas aeruginosa | ATCC 10145 | 8 | 2 | 8 | 2 | 2 | 2 | 8 | 2 | 2 | 2 |

As can be seen from Table 2, the cephalosporin compounds of the present invention possess potent and broad antibacterial activities against Gram-positive and Gram-negative bacteria as compared with the known cephalosporin antibiotics, ceftazidime.

While the invention has been described in connection with the above specific embodiments, it should be recognized that various modifications and changes as may be apparent to those skilled in the art to

which the invention pertains may be made and also fall within the scope of the invention as defined by the claims that follow.

## Claims

1. A cephalosporin compound of formula(I):

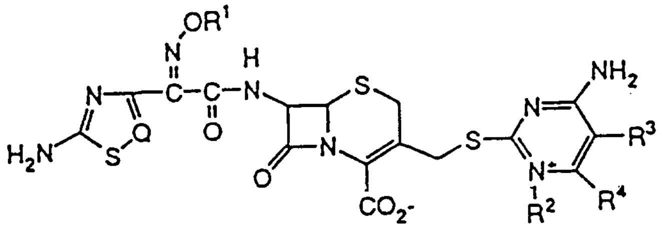

(I)

wherein:

| | |
|---|---|
| $R^1$ | is a hydrogen, a $C_{1-4}$ alkyl, $C_{3-4}$ alkenyl, $C_{3-4}$ alkynyl or $-C(R^A)(R^B)-$ COOH group wherein $R^A$ and $R^B$ are independently a hydrogen or a $C_{1-4}$ alkyl group, or form a $C_{3-7}$ cycloalkyl group together with the carbon atoms to which they are attached; |
| $R^2$ | is a $C_{1-4}$ alkyl, carboxymethyl, hydroxyethyl or amino group; |
| $R^3$ and $R^4$ | are independently a hydrogen or a $C_{1-4}$ alkyl, optionally substituted amino, benzyl, $C_{1-4}$ alkoxy or carboxymethoxy group, or form a cyclic ring, provided that $R^3$ is not a hydrogen or a $C_{1-4}$ alkyl group when $R^4$ is an unsubstituted amino group; and |
| Q | is CH or N, and a pharmaceutically acceptable non-toxic salt, physioloagically hydrolyzable ester, hydrate and solvate and isomer thereof. |

2. The compound of claim 1 wherein both $R^3$ and $R^4$ are a hydrogen or an amino group.

3. The compound of claim 2 wherein $R^1$ is a $-C(R^A)(R^B)COOH$ group where $R^A$ and $R^B$ are independently a hydrogen or a methyl or ethyl group, or form an unsubstituted $c_{4-6}$ cycloalkyl group together with the carbon atoms to which they are attached; $R^2$ is a methyl or amino group; and Q is CH.

4. The compound of claim 1 wherein the compound is selected from the group consisting of:
7-[(Z)-2-(aminothiazol-4-yl)-2-(carboxyprop-2-oxyimino) acetamido]-3-(4,5,6-triamino-1-methyl-pyrimidinium-2-yl)-thiomethyl-3-cephem-4-carboxylate;
7-[(Z)-2-(aminothiazol-4-yl)-2-(carboxyprop-2-oxyimino)acetamido]-3-(4-amino-1-methylpyrimidinium-2-yl)thiomethyl-3-cephem-4-carboxylate;
7-[(Z)-2-(aminothiazol-4-yl)-2-(ethoxyimino)acetamido]-3-(4,5,6-triamino-1-methylpyrimidinium-2-yl)-thiomethyl-3-cephem-4-carboxylate;
7-[(Z)-2-(aminothiazol-4-yl)-2-(ethoxyimino)acetamido]-3-(4-amino-1-methylpyrimidinium-2-yl)thiomethyl-3-cephem-4-carboxylate;
7-[(Z)-2-(5-amino-1,2,4-thiadiazol-3-yl)-2-(ethoxyimino)acetamido]-3-(4,5,6-triamino-1-methylpyrimidinium-2-yl)thiomethyl-3-cephem-4-carboxylate;
7-[(Z)-2-(5-amino-1,2,4-thiadiazol-3-yl)-2-(ethoxyimino)acetamido]-3-(4-amino-1-methylpyrimidinium-2-yl)thiomethyl-3-cephem-4-carboxylate;
7-[(Z)-2-(2-aminothiazol-4-yl)-2-(methoxyimino)acetamido]-3-(4,5,6-triamino-1-methylpyrimidinium-2-yl)-thiomethyl-3-cephem-4-carboxylate;
7-[(Z)-2-(2-aminothiazol-4-yl)-2-(methoxyimino)acetamido]-3-(4-amino-1-methylpyrimidinium-2-yl)-thiomethyl-3-cephem-4-carboxylate;
7-[(Z)-2-(2-aminothiazol-4-yl)-2-{(S)-1-carboxyeth-1-oxyimino}acetamido]-3-(4,5,6-triamino-1-methylpyrimidinium-2-yl)thiomethyl-3-cephem-4-carboxylate;
7-[(Z)-2-(2-aminothiazol-4-yl)-2-{(S)-1-carboxyeth-1-oxyimino}acetamido]-3-(4-amino-1-methylpyrimidinium-2-yl) thiomethyl-3-cephem-4-carboxylate;
7-[(Z)-2-(2-aminothiazol-4-yl)-2-oxyiminoacetamido]-3-(4,5,6-triamino-1-methylpyrimidinium-2-yl)-thiomethyl-3-cephem-4-carboxylate;
7-[(Z)-2-(2-aminothiazol-4-yl)-2-oxyimino)acetamido]-3-(4-amino-1-methylpyrimidinium-2-yl)thiomethyl-3-

cephem-4-carboxylate;
7-[(Z)-2-(2-aminothiazol-4-yl)-2-(carboxyprop-2-oxyimino)acetamido]-3-(4,5,6-triamino-1-ethylpyrimidinium-2-yl)-thiomethyl-3-cephem-4-carboxylate;
7-[(Z)-2-(2-aminothiazol-4-yl)-2-(carboxyprop-2-oxyimino)acetamido]-3-(4-amino-1-ethylpyrimidinium-2-yl)thiomethyl-3-cephem-4-carboxylate;
7-[(Z)-2-(2-aminothiazol-4-yl)-2-(ethoxyimino)acetamido]-3-(4,5,6-triamino-1-ethylpyrimidinium-2-yl)-thiomethyl-3-cephem-4-carboxylate;
7-[(Z)-2-(2-aminothiazol-4-yl)-2-(ethoxyimino)acetamido]-3-(4-amino-1-ethylpyrimidinium-2-yl)thiomethyl-3-cephem-4-carboxylate;
7-[(Z)-2-(5-amino-1,2,4-thiadiazol-3-yl)-2-(ethoxyimino)acetamido]-3-(4,5,6-triamino-1-ethylpyrimidinium-2-yl)-thiomethyl-3-cephem-4-carboxylate;
7-[(Z)-2-(5-amino-1,2,4-thiadiazol-3-yl)-2-(ethoxyimino)acetamido]-3-(4-amino-1-ethylpyrimidinium-2-yl)-thiomethyl-3-cephem-4-carboxylate;
7-[(Z)-2-(2-aminothiazol-4-yl)-2-(carboxyprop-2-oxyimino)acetamido]-3-(4-amino-6-methoxy-1-methylpyrimidinium-2-yl)thiomethyl-3-cephem-4-carboxylate;
7-[(Z)-2-(2-aminothiazol-4-yl)-2-(ethoxyimino)acetamido]-3-(4-amino-6-methoxy-1-methylpyrimidinium-2-yl)thiomethyl-3-cephem-4-carboxylate;
7-[(Z)-2-(2-aminothiazol-4-yl)-2-(ethoxyimino)acetamido]-3-(4-amino-6-carboxymethoxy-1-methylpyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-6-phenyl-1-methylpyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-5-phenyl-1-methylpyrimidinium-2-yl)-thiomethyl-3-cephem-4-carboxylate;
7-[(Z)-2-(2-aminothiazol-4-yl)-2-(methoxyimino)acetamido]-3-(4-amino-1-carboxymethylpyrimidinium-2-yl)thiomethyl-3-cephem-4-carboxylate;
7-[(Z)-2-(2-aminothiazol-4-yl)-2-(ethoxyimino)acetamido]-3-(4-amino-1-carboxymethylpyrimidinium-2-yl)-thiomethyl-3-cephem-4-carboxylate;
7-[(Z)-2-(2-aminothiazol-4-yl)-2-(carboxyprop-2-oxyimino)acetamido]-3-(4-amino-1-carboxymethylpyrimidinium-2-yl)-thiomethyl-3-cephem-4-carboxylate;
7-[(Z)-2-(2-aminothiazol-4-yl)-2-(ethoxyimino)acetamido]-3-(4-amino-5-carboxy-1-methylpyrimidinium-2-yl)thiomethyl-3-cephem-4-carboxylate;
7-[(Z)-2-(2-aminothiazol-4-yl)-2-(ethoxyimino)acetamido]-3-(4,5-diamino-1-methylpyrimidinium-2-yl)-thiomethyl-3-cephem-4-carboxylate;
7-[(Z)-2-(2-aminothiazol-4-yl)-2-(carboxyprop-2-oxyimino)acetamido]-3-(4,5-diamino-1-methylpyrimidinium-2-yl)-thiomethyl-3-cephem-4-carboxylate;
7-[(Z)-2-(2-aminothiazol-4-yl)-2-(carboxyprop-2-oxyimino)acetamido]-3-(4-amino-1-methylbenzo[5,6,e]-pyrimidinium-2-yl)thiomethyl-3-cephem-4-carboxylate;
7-[(Z)-2-(2-aminothiazol-4-yl)-2-(ethoxyimino)acetamido]-3-(4-amino-1-methylbenzo[5,6,e]pyrimidinium-2-yl)thiomethyl-3-cephem-4-carboxylate;
7-[(Z)-2-(2-aminothiazol-4-yl)-2-(carboxyprop-2-oxyimino)acetamido]-3-(6-amino-3-methylpyrido[5,6,e]-pyrimidinium-4-yl)thiomethyl-3-cephem-4-carboxylate;
7-[(Z)-2-(2-aminothiazol-4-yl)-2-(ethoxyimino)acetamido]-3-(6-amino-3-methylpyrido[5,6,e]pyrimidinium-4-yl)thiomethyl-3-cephem-4-carboxylate;
7-[(Z)-2-(2-aminothiazol-4-yl)-2-(carboxyprop-2-oxyimino)acetamido]-3-(4-amino-1-methylfuro[5,6,e]-pyrimidinium-2-yl)thiomethyl-3-cephem-4-carboxylate;
7-[(Z)-2-(2-aminothiazol-4-yl)-2-(ethoxyimino)acetamido]-3-(4-amino-1-methylfuro[5,6,e]pyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-5-benzyl-1-methylpyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-6-methylamino-1-methylpyrimidinium-2-yl)thiomethyl-3-cephem-4-carboxylate;
7-[(Z)-2-(2-aminothiazol-4-yl)-2-(ethoxyimino)acetamido]-3-(4-amino-6-methylamino-1-methylpyrimidinium-2-yl)thiomethyl-3-cephem-4-carboxylate;
7-[(Z)-2-(2-aminothiazol-4-yl )-2-(carboxyprop-2-oxyimino)acetamido]-3-(4-amino-6-ethylamino-1-methyl-pyrimidinium-2-yl)thiomethyl-3-cephem-4-carboxylate;
7-[(Z)-2-(2-aminothiazol-4-yl)-2-(ethoxyimino)acetamido]-3-(4-amino-6-ethylamino-1-methylpyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-5-carboxymethylpyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-5-formamido-1-methylpyrimidinium-2-yl)thiomethyl-3-cephem-4-carboxylate;
7-[(Z)-2-(2-aminothiazol-4-yl)-2-(ethoxyimino)acetamido]-3-(4-amino-5-formamido-1-methylpyrimidinium-2-yl)thiomethyl-3-cephem-4-carboxylate;
7-[(Z)-2-(2-aminothiazol-4-yl)-2-(ethoxyimino)acetamido]-3-(1,4-diaminopyrimidinium-2-yl)thiomethyl-3-cephem-4-carboxylate;
7-[(Z)-2-(2-aminothiazol-4-yl)-2-(carboxyprop-2-oxyimino)acetamido]-3-(1,4-diaminopyrimidinium-2-yl)-thiomethyl-3-cephem-4-carboxylate;
7-[(Z)-2-(2-aminothiazol-4-yl)-2-{(S)-1-carboxyeth-1-oxyimino}acetamido]-3-(1,4-diaminopyrimidinium-2-yl)thiomethyl-3-cephem-4-carboxylate;
7-[(Z)-2-(2-aminothiazol-4-yl)-2-(methoxyimino)acetamido]-3-(1,4-diaminopyrimidinium-2-yl)thiomethyl-3-cephem-4-carboxylate;
7-[(Z)-2-(5-amino-1,2,4-thiadiazol-3-yl)-2-(ethoxyimino)acetamido]-3-(1,4-diaminopyrimidinium-2-yl)-thiomethyl-3-cephem-4-carboxylate;
7-[(Z)-2-(2-aminothiazol-4-yl)-2-(ethoxyimino)acetamido]-3-(1,4,5-triaminopyrimidinium-2-yl)thiomethyl-3-cephem-4-carboxylate;
7-[(Z)-2-(2-aminothiazol-4-yl)-2-(carboxyprop-2-oxyimino)acetamido]-3-(1,4,5-triaminopyrimidinium-2-yl)-thiomethyl-3-cephem-4-carboxylate;
7-[(Z)-2-(2-aminothiazol-4-yl)-2-(methoxyimino)acetamido]-3-(1,4,5-triaminopyrimidinium-2-yl)thiomethyl-3-cephem-4-carboxylate;
7-[(Z)-2-(5-amino-1,2,4-thiadiazol-3-yl)-2-(ethoxyimino)acetamido]-3-(1,4,5-triaminopyrimidinium-2-yl)-thiomethyl-3-cephem-4-carboxylate;
7-[(Z)-2-(2-aminothiazol-4-yl)-2-{(R)-1-carboxyeth-1-oxyimino} acetamido]-3-(4,5,6-triamino-1-methyl-pyrimidinium-2-yl)thiomethyl-3-cephem-4-carboxylate;
7-[(Z)-2-(2-aminothiazol-4-yl)-2-{(R)-1-carboxyeth-1-oxyimino}acetamido]-3-(4-amino-1-methylpyrimidinium-2-yl)thiomethyl-3-cephem-4-carboxylate;
7-[(Z)-2-(2-aminothiazol-4-yl)-2-{(R)-1-carboxyeth-1-oxyimino} acetamido]-3-(1,4-diaminopyrimidinium-2-yl)thiomethyl-3-cephem-4-carboxylate;
7-[(Z)-2-(2-aminothiazol-4-yl)-2-(carboxyprop-2-oxyimino)acetamido]-3-(4-amino-1-methyl-6-(N,N-dimethyl)aminopyrimidinium-2-yl}thiomethyl-3-cephem-4-carboxylate;
7-[(Z)-2-(2-aminothiazol-4-yl)-2-(methoxyimino)acetamido]-3-{4-amino-1-methyl-6-(N,N-dimethyl)-aminopyrimidinium-2-yl}thiomethyl-3-cephem-4-carboxylate;
7-[(Z)-2-(2-aminothiazol-4-yl)-2-(ethoxyimino)acetamido]-3-{4-amino-1-methyl-6-(N,N-dimethyl)-aminopyrimidinium-2-yl}thiomethyl-3-cephem-4-carboxylate;
7-[(Z)-2-(2-aminothiazol-4-yl)-2-(carboxyprop-2-oxyimino)acetamido]-3-(1,4,5,6-tetraaminopyrimidinium-2-yl)thiomethyl-3-cephem-4-carboxylate;
7-[(Z)-2-(2-aminothiazol-4-yl)-2-(methoxyimino)acetamido]-3-(1,4,5,6-tetraaminopyrimidinium-2-yl)-thiomethyl-3-cephem-4-carboxylate;
7-[(Z)-2-(2-aminothiazol-4-yl)-2-(ethoxyimino)acetamido]-3-(1,4,5,6-tetraaminopyrimidinium-2-yl)-thiomethyl-3-cephem-4-carboxylate;
7-[(Z)-2-(5-amino-1,2,4-thiadiazol-3-yl)-2-(ethoxyimino)acetamido]-3-(1,4,5,6-tetraaminopyrimidinium-2-yl)thiomethyl-3-cephem-4-carboxylate;
7-[(Z)-2-(2-aminothiazol-4-yl)-2-(carboxyprop-2-oxyimino)acetamido]-3-(1,4-diamino-5-methylpyrimidinium-2-yl)thiomethyl-3-cephem-4-carboxylate;
7-[(Z)-2-(2-aminothiazol-4-yl)-2-{(S)-1-carboxyeth-1-oxyimino}acetamido]-3-(1,4-diamino-5-methylpyrimidinium-2-yl)thiomethyl-3-cephem-4-carboxylate;
7-[(Z)-2-(2-aminothiazol-4-yl)-2-(carboxymethoxyimino)acetamido]-3-(1,4-diamino-5-methylpyrimidinium-2-yl)thiomethyl-3-cephem-4-carboxylate;
7-[(Z)-2-(2-aminothiazol-4-yl)-2-(methoxyimino)acetamido]-3-(1,4-diamino-5-methylpyrimidinium-2-yl)-thiomethyl-3-cephem-4-carboxylate;
7-[(Z)-2-(2-aminothiazol-4-yl)-2-(ethoxyimino)acetamido]-3-(1,4-diamino-5-methylpyrimidinium-2-yl)-thiomethyl-3-cephem-4-carboxylate;
7-[(Z)-2-(5-amino-1,2,4-thiadiazol-3-yl)-2-(ethoxyimino)acetamido]-3-(1,4-diamino-5-methylpyrimidinium-2-yl)thiomethyl-3-cephem-4-carboxylate;
7-[(Z)-2-(2-aminothiazol-4-yl)-2-(carboxyprop-2-oxyimino)acetamido]-3-(1,4-diamino-5-ethylpyrimidinium-2-yl)thiomethyl-3-cephem-4-carboxylate;
7-[(Z)-2-(2-aminothiazol-4-yl)-2-(ethoxyimino)acetamido]-3-(1,4-diamino-5-ethylpyrimidinium-2-yl)-

thiomethyl-3-cephem-4-carboxylate; 7-[(Z)-2-(2-aminothiazol-4-yl)-2-(methoxyimino)acetamido]-3-(1,4-diamino-5-ethylpyrimidinium-2-yl)-thiomethyl-3-cephem-4-carboxylate; 7-[(Z)-2-(2-aminothiazol-4-yl)-2-(carboxyprop-2-oxyimino)acetamido]-3-{1,4-diamino-6-(N-methyl)-aminopyrimidinium-2-yl)thiomethyl-3-cephem-4-carboxylate; 7-[(Z)-2-(2-aminothiazol-4-yl)-2-(ethoxyimino)-acetamido]-3-{1,4-diamino-6-(N-methyl)-aminopyrimidinium-2-yl}thiomethyl-3-cephem-4-carboxylate; 7-[(Z)-2-(2-aminothiazol-4-yl)-2-(methoxyimino)acetamido]-3-{1,4-diamino-6-(N-methyl)-aminopyrimidinium-2-yl}thiomethyl-3-cephem-4-carboxylate; 7-[(Z)-2-(5-amino-1,2,4-thiadiazol-3-yl)-2-(ethoxyimino)acetamido]-3-(1,4-diamino-6-(N-methyl)-aminopyrimidinium-2-yl}thiomethyl-3-cephem-4-carboxylate; 7-[(Z)-2-(2-aminothiazol-4-yl)-2-{(S)-1-carboxyeth-1-oxyimino}acetamido]-3-{1,4-diamino-6-(N-methyl)-aminopyrimidinium-2-yl}thiomethyl-3-cephem-4-carboxylate; 7-[(Z)-2-(2-aminothiazol-4-yl)-2-(carboxymethoxyimino)acetamido]-3-{1,4-diamino-6-(N-methyl)-aminopyrimidinium-2-yl)thiomethyl-3-cephem-4-carboxylate; 7-[(Z)-2-(2-aminothiazol-4-yl)-2-(carboxyprop-2-oxyimino)acetamido]-3-{1,4-diamino-5-methyl-6-(N-methyl)aminopyrimidinium-2-yl}thiomethyl-3-cephem-4-carboxylate; 7-[(Z)-2-(2-aminothiazol-4-yl)-2-(ethoxyimino)acetamido]-3-{1,4-diamino-5-methyl-6-(N-methyl)-aminopyrimidinium-2-yl}thiomethyl-3-cephem-4-carboxylate; 7-[(Z)-2-(2-aminothiazol-4-yl)-2-(methoxyimino)acetamido]-3-{1,4-diamino-5-methyl-6-(N-methyl)-aminopyrimidinium-2-yl}thiomethyl-3-cephem-4-carboxylate; 7-[(Z)-2-(5-amino-1,2,4-thiadiazol-3-yl)-2-(ethoxyimino)acetamido]-3-{1,4-diamino-5-methyl-6-(N-methyl)-aminopyrimidinium-2-yl}thiomethyl-3-cephem-4-carboxylate; 7-[(Z)-2-(2-aminothiazol-4-yl)-2-{(S)-1-carboxyeth-1-oxyimino}acetamido]-3-{1,4-diamino-5-methyl-6-(N-methyl)aminopyrimidinium-2-yl}thiomethyl-3-cephem-4-carboxylate; 7-[(Z)-2-(2-aminothiazol-4-yl)-2-(carboxymethoxyimino)acetamido]-3-{1,4-diamino-5-methyl-6-(N-methyl)aminopyrimidinium-2-yl}thiomethyl-3-cephem-4-carboxylate; 7-[(Z)-2-(2-aminothiazol-4-yl)-2-(carboxymethoxyimino)acetamido]-3-(1,4-diaminopyrimidinium-2-yl)-thiomethyl-3-cephem-4-carboxylate; 7-[(Z)-2-(2-aminothiazol-4-yl)-2-(carboxycyclobutoxyimino)acetamido]-3-(1,4-diaminopyrimidinium-2-yl)-thiomethyl-3-cephem-4-carboxylate; 7-[(Z)-2-(2-aminothiazol-4-yl)-2-(carboxycyclobutoxyimino)acetamido]-3-(4-amino-1-methylpyrimidinium-2-yl)thiomethyl-3-cephem-4-carboxylate; 7-[(Z)-2-(2-aminothiazol-4-yl)-2-(carboxycyclopentoxyimino)acetamido]-3-(1,4-diaminopyrimidinium-2-yl)-thiomethyl-3-cephem-4-carboxylate; 7-[(Z)-2-(2-aminothiazol-4-yl)-2-(carboxycyclopentoxyimino)acetamido]-3-(4-amino-1-methylpyrimidinium-2-yl)thiomethyl-3-cephem-4-carboxylate; 7-[(Z)-2-(2-aminothiazol-4-yl)-2-(carboxycyclohexoxyimino)acetamido]-3-(1,4-diaminopyrimidinium-2-yl)-thiomethyl-3-cephem-4-carboxylate; 7-[(Z)-2-(2-aminothiazol-4-yl)-2-(carboxycyclohexoxyimino)acetamido]-3-(4-amino-1-methylpyrimidinium-2-yl)thiomethyl-3-cephem-4-carboxylate; 7-[(Z)-2-(2-aminothiazol-4-yl)-2-{(S)-1-carboxyprop-1-oxyimino}acetamido]-3-(1,4-diaminopyrimidinium-2-yl)thiomethyl-3-cephem-4-carboxylate; 7-[(Z)-2-(2-aminothiazol-4-yl)-2-{(R)-1-carboxyprop-1-oxyimino}acetamido]-3-(1,4-diaminopyrimidinium-2-yl)thiomethyl-3-cephem-4-carboxylate; 7-[(Z)-2-(2-aminothiazol-4-yl)-2-{(S)-1-carboxyprop-1-oxyimino}acetamido]-3-(4-amino-1-methylpyrimidinium-2-yl)thiomethyl-3-cephem-4-carboxylate; 7-[(Z)-2-(2-aminothiazol-4-yl)-2-{(R)-1-carboxyprop-1-oxyimino}acetamido]-3-(4-amino-1-methylpyrimidinium-2-yl)thiomethyl-3-cephem-4-carboxylate); 7-[(Z)-2-(2-aminothiazol-4-yl)-2-{(R)-2-carboxybut-2-oxyimino}acetamido]-3-(1,4-diaminopyrimidinium-2-yl)thiomethyl-3-cephem-4-carboxylate; and 7-[(Z)-2-(2-aminothiazol-4-yl)-2-{(R)-2-carboxybut-2-oxyimino)acetamido]-3-(4-amino-1-methylpyrimidinium-2-yl)thiomethyl-3-cephem-4-carboxylate.

**5.** A pharmaceutical composition comprising an effective amount of th cephalosporin compound defined in claim 1 as an active ingredient, and a pharmaceutically acceptable carrier.